# EUROPÄISCHE PATENTSCHRIFT

(11) **EP 0 474 817 B1**
(45) Veröffentlichungstag und Bekanntmachung des Hinweises auf die Patenterteilung: **11.10.1995**
(21) Anmeldenummer: 91906405.5
(22) Anmeldetag: 27.03.1991
(51) Int. Cl.: C07C 405/00, A61K 31/557

(54) **BICYCLO [3.3.0]OCTAN-DERIVATE, VERFAHREN ZU IHRER HERSTELLUNG UND IHRE PHARMAZEUTISCHE VERWENDUNG**
BICYCLO [3.3.0]OCTANE DERIVATIVES, PROCESS FOR PRODUCING THEM AND THEIR PHARMACEUTICAL USE
DERIVES DE BICYCLO [3.3.0]OCTANE, LEUR PROCEDE DE PRODUCTION ET LEUR UTILISATION PHARMACEUTIQUE

(30) Priorität: 28.03.1990 DE 4010355
(43) Veröffentlichungstag der Anmeldung: 18.03.1992
(73) Patentinhaber: SCHERING AKTIENGESELLSCHAFT, 13342 Berlin (DE)
(72) Erfinder: KLAR, Ulrich, D-1000 Berlin 27 (DE); REHWINKEL, Hartmut, D-1000 Berlin 44 (DE); VORBRÜGGEN, Helmut, D-1000 Berlin 27 (DE); THIERAUCH, Karl-Heinz, D-1000 Berlin 37 (DE); VERHALLEN, Peter, D-1000 Berlin 28 (DE)
(86) Internationale Anmeldenummer: DE9100278
(87) Internationale Veröffentlichungsnummer: WO9114675

(56) Entgegenhaltungen:
- EP-A- 0 011 591
- EP-A- 0 231 078
- WO-A-82/00142

## Beschreibung

Die Erfindung betrifft Bicyclo[3.3.0]octan-Derivate, Verfahren zu ihrer Herstellung sowie ihre Verwendung als Hilfsstoffe für pharmakologische Untersuchungen und als Arzneimittel.
Bicyclo[3.3.0]octan-Derivate sind in den letzten Jahren intensiv bearbeitet worden, da vom Bicyclo[3.3.0]octan-System abgeleitete Carbacycline wie z.B. Iloprost bzw. Cicaprost oder andere analoge Isocarbacycline biologisch sehr potente wie auch chemisch und teils auch metabolisch stabile Prostacyclin-Mimetika darstellen.
Es wurde überraschenderweise gefunden, daß durch die Einführung eines Stickstoffatomes in Position 13 oder 14 (Prostaglandin-Zählweise) des Carbacyclingerüstes chemisch und metabolisch stabile Carbacyclin-Analoga erhalten werden, die in der Lage sind, die pharmakologischen Eigenschaften des instabilen Thomboxan-A₂ (TXA₂) bzw. PGH₂ sowie seinen stabilen Analoga wie z.B. U46619 oder U44069 zu antagonisieren. Darüber hinaus kann durch die Wahl der Lage bzw. Konfiguration einer Doppelbindung zwischen den mit a bis d gekennzeichneten Kohlenstoffatomen eine zusätzliche, dem chemisch und metabolisch sehr instabilen Prostacyclin (PGI₂) vergleichbare agonistische Wirkkomponente eingeführt werden.
Die Verbindungen dieser Erfindung stellen deshalb wertvolle Hilfsmittel zur selektiven Therapie von Erkrankungen, die auf einem Mangel an körpereigenem PGI₂ und/oder Überschuß an TXA₂ bzw. PGH₂ zurückzuführen sind, dar.
Die Erfindung betrifft Bicyclo[3.3.0]octan-Derivate der Formel I, 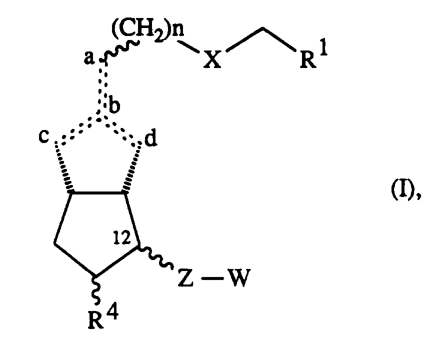
sowie deren Enantiomere,
worin
zwischen den Kohlenstoffatomen der Zentren a-b oder b-c oder b-d maximal eine Doppelbindung liegt, 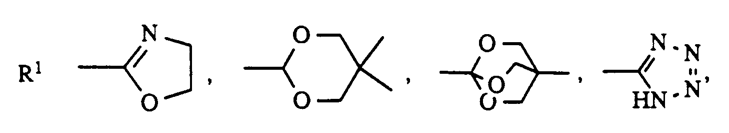
COOR⁵, wobei R⁵
Wasserstoff oder gegebenenfalls durch Halogen, Phenyl, C₁-C₄-Alkoxy oder Di-(C₁-C₄)-alkylamino substituiertes C₁-C₁₀-Alkyl, C₃-C₁₀-Cycloalkyl, C₇-C₁₆-Aralkyl, durch Y substituiertes Phenacyl oder C₆-C₁₂-Aryl oder einen 5- oder 6- gliedrigen heterocyclischen Rest mit wenigstens einem N-, O- oder S-Atom bedeuten kann, oder -CONHR⁷ mit R⁷ in der Bedeutung Wasserstoff, C₁-C₁₀-Alkanoyl oder C₁-C₁₀-Alkansulfonyl sein kann,
X eine CH₂-Gruppe oder ein O- oder S-Atom,
n 0-2,
R⁴ ein Wasserstoffatom, eine freie oder funktionell abgewandelte Hydroxygruppe, wobei die OH-Gruppe α- oder β-ständig sein kann, 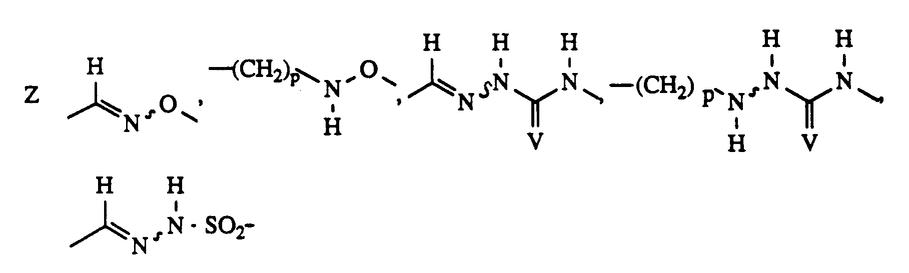
p 0 oder 1,
V ein O- oder S-Atom,
W durch Y substituiertes C₁-C₁₀-Alkyl, C₃-C₁₀-Cycloalkyl, 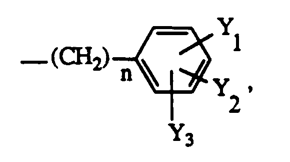 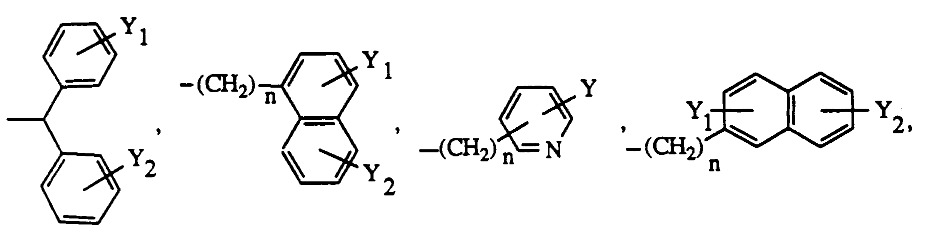 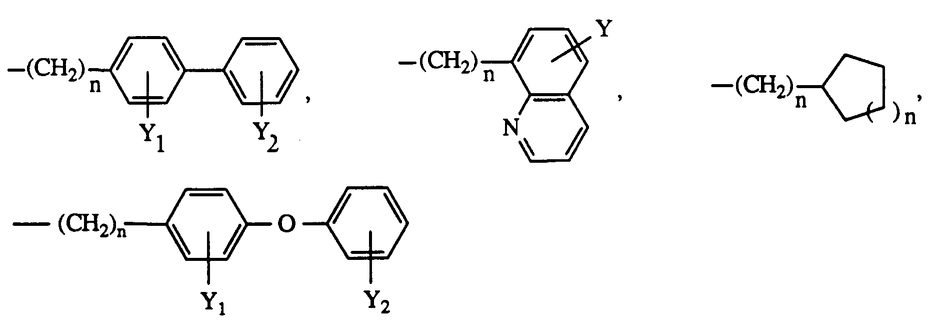
Y₁, Y₂ und Y₃ gleich oder verschieden sind und Y bedeuten,
Y Wasserstoff, Halogen, N₃, CF₃, OR⁶, NO₂, COOR⁶ oder C₁-C₁₀-Alkyl,
R⁶ Wasserstoff, C₁-C₁₀-Alkyl, gegebenenfalls durch Halogen substituiertes C₆-C₁₂-Aryl oder C₇-C₁₆-Aralkyl sein kann und, falls R⁵ Wasserstoff bedeutet, deren Salze mit physiologisch vertraglichen Basen, sowie die α-, β- oder γ-Cyclodextrinclathrate, sowie die mit Liposomen verkapselten Verbindungen der Formel I bedeuten.
Die Definition 5- oder 6-gliedriger heterocyclischer Rest betrifft Heterocyclen, die wenigstens ein Heteroatom, vorzugsweise Stickstoff, Sauerstoff oder Schwefel enthalten. Beispielsweise seien genannt 2-Furyl, 3-Furyl, 2-Thienyl, 3-Thienyl, 2-Pyridyl, 3-Pyridyl, 4-Pyridyl.
Als Alkylgruppen R⁵, R⁶, W und Y sind gerad- oder verzweigtkettige Alkylgruppen mit 1 - 10 C-Atomen zu bettachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.
Die Alkylgruppen R⁵, R⁶, W und Y können substituiert sein durch Halogenatome, Hydroxygruppen, C₁-C₄-Alkoxygruppen, C₆-C₁₂-Arylgruppen, die durch Halogen substituiert sein können, Di-(C₁-C₄)-Alkylamine und Tri-(C₁-C₄)-Alkylammonium Bevorzugt sind solche Alkylgruppen, die einfach substituiert sind.
Als Substituenten seien beispielsweise genannt Fluor-, Chlor- oder Bromatome, Phenyl, Dimethylamino, Diethylamino, Methoxy, Ethoxy.
Als bevorzugte Alkylgruppen R⁵, R⁶, W und Y sind solche mit 1 - 4 C-Atomen, wie z. B. Methyl, Ethyl, Propyl, Isobutyl, Butyl, zu nennen.
Als Arylgruppen R⁵ und R⁶ kommen beispielsweise in Betracht: Phenyl, Diphenyl, 1-Naphthyl und 2-Naphthyl, die substituiert sein können durch 1 - 3 Halogenatome, eine Phenylgruppe, 1 - 3 Alkylgruppen mit jeweils 1 - 4 C-Atomen eine Chlormethyl-, Fluormethyl-, Carboxyl-, C₁-C₄-Alkoxy- oder Hydroxygruppe. Bevorzugt ist die Substitution in 3- und 4-Stellung am Phenylring zum Beispiel durch Fluor, Chlor, C₁-C₄- Alkoxy oder Trifluormethyl oder in 4-Stellung durch Hydroxy.
Die Cycloalkylgruppen R⁵ und W können im Ring 3-10, vorzugsweise 3-6 Kohlenstoffatome enthalten. Die Ringe können durch Alkylgruppen mit 1-4 Kohlenstoffatomen substituiert sein. Beispielsweise seien genannt Cyclopropyl, Cyclobutyl, Cyclopentyl, Cyclohexyl, Methylcyclopentyl, Methylcyclohexyl.
Die C₇-C₁₆ Aralkylgruppen in R¹ können im Ring 6 bis 14 C-Atome enthalten, bevorzugt 6 bis 10 (Phenyl oder Naphtyl) und in der Alkylkette 1 bis 4, bevorzugt 1 bis 2 C-Atome. Bevorzugte Aralkylreste sind z.B. Benzyl, Phenylethyl, 1-Phenylethyl, 1-(2)-Naphthylmethyl bzw. 1-(2)-Naphthylethyl.
Die unter den Definitionen genannten C₁-C₁₀-Alkylgruppen sollen geradkettige oder verzweigte Alkylgruppen sein, wie sie für die vorstehenden Alkylgruppen bereits genannt wurden.
Die Hydroxygruppen in R⁴ und W können funktionell abgewandelt sein, beispielsweise durch Veretherung oder Veresterung, wobei die freie oder abgewandelte Hydroxygruppe in R⁴ α- oder β-ständig sein kann, wobei freie Hydroxygruppen bevorzugt sind.
Als Ether- und Acylreste kommen die dem Fachmann bekannten Reste in Betracht.
Bevorzugt sind leicht abspaltbare Etherreste, wie beispielsweise der Tetrahydropyranyl-, Tetrahydrofuranyl-, tert-Butyldimethylsilyl-, tert-Butyldiphenylsilyl-, Tribenzylsilylrest. Als Acylreste kommen z.B. Acetyl, Propionyl, Butyryl, Benzoyl in Frage.
Halogen in den Definitionen für R⁵, R⁶ und Y bedeutet Fluor, Chlor, Brom und Iod.
Die Reste "C₁-C₁₀-Alkanoyl" oder "C₁-C₁₀-Alkansulfonyl" für R⁷ entsprechen den bereits genannten Alkylgruppen gleicher Läge mit dem Unterschied, daß sie an eine Carboxylgruppe gebunden sind. Bevorzugt sind C₁-C₄-Alkanoyl bzw. -Alkansulfonyl.
Zur Salzbildung mit den freien Säuren (R⁵ = H) sind anorganische und organische Basen geeignet, wie sie dem Fachmann zur Bildung physiologisch verträglicher Salze bekannt sind Beispielsweise seien genannt: Alkalihydroxide, wie Natrium- oder Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin u.s.w.

Bevorzugt werden die Verbindungen der Formel I, in denen
- R¹: die Gruppen COOR⁵,
- R⁴: Wasserstoff oder Hydroxyl,
- R⁵: Wasserstoff oder Methyl,
- R⁷: Methansulfonyl,
- X: Sauerstoff oder CH₂,
- n: 0 oder 1,
- V: Sauerstoff.

Die Erfindung betrifft ferner ein Verfahren zur Herstellung der Verbindungen der Formel I, das dadurch gekennzeichnet ist, daß man eine Verbindung der Formel II 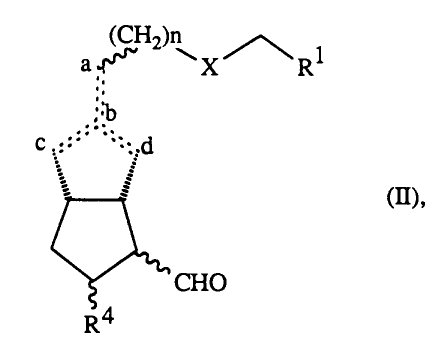
worin R¹, X, n, a-b, b-c, b-d und R⁴ die oben angegebenen Bedeutungen aufweisen und freie OH-Gruppen in R⁴ geschützt sind, mit Aminoverbindungen der Formel 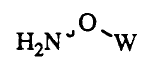
oder 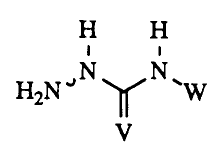
(bzw. des Hydrochlorides) oder H₂N-NH-SO₂-W worin V und W die oben angegebenen Bedeutungen aufweisen und freie OH-Gruppen in W geschützt sind umsetzt und gegebenenfalls geschützte Hydroxygruppen in R⁴ und W freisetzt und/oder freie Hydroxygruppen verestert, verethert und/oder eine veresterte Carboxygruppe verseift oder eine Carboxygruppe mit einer physiologisch verträglichen Base in ein Salz überführt oder mit α-, β- oder γ-Cyclodextrin zu einem Clathrat umsetzt oder mit Liposomen verkapselt.
Die Umsetzung der Verbindungen der allgemeinen Formel II zu den Verbindungen der allgemeinen Formel I wird mit den oben genannten Aminoverbindungen in alkohotischer Lösung (bevorzugt ethanolischer) in Gegenwart katalytischer (equimolarer) Mengen einer organischen Base (z.B. Pyridin, DBN, DBU, Triethylamin, DMAP usw.) bei 20-100°C (bevorzugt 40-60°C) in 2-24 Stunden (bevorzugt 2-10 Stunden) durchgeführt.
Die Freisetzung der funktionell abgewandelten Hydroxygruppen R⁴ und W erfolgt nach den dem Fachmann bekannten Methoden. Beispielsweise wird die Abspaltung von Etherschutzgruppen in einer wässrigen Lösung einer organischen Säure, wie z.B. Essigsäure, Propionsäure, Zitronensäure u.a. oder in einer wässrigen Lösung einer anorganischen Säure, wie z.B. Salzsäure, oder im Falle von Tetrahydropyranylethern unter Verwendung von Pyrridinium-p-Toluolsulfonat, vorzugsweise in Alkoholen als Lösungsmittel oder unter Verwendung von wasserfreiem Magnesiumbromid, vorzugsweise in Diethylether als Lösungsmittel durchgeführt.

Zur Verbesserung der Löslichkeit wird bei Verwendung wässrig-saurer Reaktionsbedingungen zweckmäßigerweise ein mit Wasser mischbares inertes Lösungsmittel zugesetzt. Als geeignet erweisen sich z.B. Alkohole wie Methanol und Ethanol, Ether wie Dimethoxyethan, Dioxan und Tetrahydrofuran, wobei Tetrahydrofuran bevorzugt angewendet wird.

Die Abspaltung der Silyletherschutzgruppen erfolgt beispielsweise mit Tetraburylammoniumfluorid nach den dem Fachmann bekannten Methoden. Als Lösungsmittel sind beispielsweise Tetrahydrofuran, Diethylether, Dioxan, Methylenchlorid etc. geeignet. Die Abspaltung wird vorzugsweise bei Temperaturen zwischen 20°C und 80°C durchgeführt.

Die Verseifung der Acylgruppen und Carbacyclinester wird nach den dem Fachmann bekannten Methoden durchgeführt, wie beispielsweise mit basischen Katalysatoren wie z.B. mit Alkali- oder Erdalkali-carbonaten oder -hydroxiden in einem Alkohol oder der wässrigen Lösung eines Alkohols. Als Alkohole kommen aliphatische Alkohole wie z.B. Methanol, Ethanol, Butanol etc. in Betracht, vorzugsweise jedoch Methanol. Als Alkalicarbonate und -hydroxide seien Lithium-, Natrium- und Kaliumsalze genannt Bevorzugt sind die Lithium- und Kaliumsalze. Als Erdalkalicarbonate und -hydroxide eignen sich beispielsweise Calciumcarbonat, Calciumhydroxid und Bariumcarbonat. Die Umsetzung erfolgt allgemein bei -10°C bis +70°C, vorzugsweise jedoch bei +25°C.

Die Einführung der Estergruppe CO₂R⁵ für R¹ bzw. CO₂R⁶ für Y, bei welcher R⁵ bzw. R⁶ eine Alkylgruppe mit 1-10-C-Atomen darstellt, erfolgt nach den dem Fachmann bekannten Methoden. Die Carboxyverbindungen (R⁵ = H bzw. R⁶ = H) werden beispielsweise mit Diazokohlenwasserstoffen in an sich bekannter Weise umgesetzt. Die Veresterung mit Diazokohlenwasserstoffen erfolgt z.B. dadurch, daß eine Lösung des Diazokohlenwasserstoffes in einem inerten Lösungsmittel, vorzugsweise in Diethylether, mit der Carboxyverbindung, gelöst in dem gleichen oder in einem anderen ebenfalls inerten Lösungsmittel, wie z.B. Methylenchlorid, vermischt wird. Nach beendeter Umsetzung in 1 bis 60 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389-394 (1954)].

Die Einführung der Estergruppe CO₂R⁵ für R¹ bzw. CO₂R⁶ für Y, bei welcher R⁵ bzw. R⁶ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base wie z.B. Pryridin, DMAP, Triethylamin, in einem inerten Lösungsmittel wie z.B. Methylenchlorid, Ethylenchlorid, Chloroform, Essigsäureethylester, Tetrahydrofuran, vorzugsweise jedoch mit Chloroform umgesetzt. Die Reaktion wird bei Temperaturen zwischen -30°C und +50°C, vorzugsweise bei +10°C, durchgeführt.

Die Carbacyclinderivate der Formel I mit R⁵ bzw. R⁶ in der Bedeutung eines Wasserstoffatomes können mit geeigneten Mengen der entsprechenden anorganischen Basen unter Neutralisierung in Salze überführt werden. Beispielsweise erhält man beim Lösen der entsprechenden Säuren in Wasser, welches stöchiometrische Mengen der Base enthält, nach Abdampfen des Wassers oder nach Zugabe eines mit Wasser mischbaren Lösungsmittels, z.B. Alkohol oder Aceton, das feste anorganische Salz.

Die Herstellung der Aminsalze erfolgt in üblicher Weise. Dazu löst man die Säure in einem geeigneten Lösungsmittel, wie z.B. Ethanol, Aceton, Diethylether oder Benzol und setzt 1 bis 5 Äquivalente des jeweiligen Amins dieser Lösung zu. Dabei fällt das Salz gewöhnlich in fester Form an oder wird nach Verdampfen des Lösungsmittels in üblicher Weise isoliert.

Die funktionelle Abwandlung der freien Hydroxygruppen erfolgt nach den dem Fachmann bekannten Methoden. Zur Einführung der Etherschutzgruppen wird beispielsweise mit Dihydropyran oder Methylvinylether in Methylenchlorid oder Chloroform unter Verwendung katalytischer Mengen eines sauren Kondensationsmittels wie z.B. p-Toluolsulfonsäure, umgesetzt. Der jeweilige Enolether wird im Überschuß, vorzugsweise in der 1,2- bis 10-fachen Menge des theoretischen Bedarfs, zugesetzt. Die Umsetzung erfolgt normalerweise bei -10°C bis +30°C und ist nach 2 bis 45 Minuten beendet.

Zur Einführung von Silyletherschutzgruppen wird beispielsweise mit t-Butyl-diphenylchlorsilan oder t-Butyl-dimethylchlorsilan in Dimethylformamid unter Verwendung einer Base wie z.B. Imidazol, umgesetzt. Das jeweilige Silylchlorid wird im Überschuß, vorzugsweise in der 1,05- bis 4-fachen Menge des theoretischen Bedarfs, zugesetzt. Die Umsetzung erfolgt normalerweise bei 0°C bis 30°C und ist nach 1 bis 24 Stunden beendet.

Die Einführung der Acylschutzgruppen erfolgt, indem man eine Verbindung der Formel I in an sich bekannter Weise mit einem Carbonsäurederivat, wie z.B. Säurechlorid, Säureanhydrid etc., umsetzt.

### Biologische Wirkung und Anwendungsbereich der neuen TXA₂-Antagonisten:

Die Verbindungen dieser Erfindung eignen sich zur Therapie von Erkrankungen des cardiovaskulären Systems, des Magens, des Pankreas, der Leber und der Niere. Sie wirken blutdrucksenkend und bronchodilatorisch. Sie sind hervorragend geeignet zur Hemmung der Thrombozytenaktivierung. Folglich stellen die neuen TXA₂-Antagonisten der Formel I wertvolle pharmazeutische Wirkstoffe dar. Darüber hinaus zeichnen sich die Verbindungen durch die zusätzliche, gezielt einbringbare PGI₂-agonistische Wirkqualität und einem hierdurch bedingten breiteren Anwendungsspektrum, durch höhere Selektivität (bei fehlendem TXA₂-Partialagonismus), einer wesentlich längeren Wirksamkeit und einer größeren Stabilität verglichen mit ähnlichen TXA₂-Antagonisten aus.
Die neuen TXA₂-Antagonisten besitzen die für diese Verbindungsklasse typischen Eigenschaften, wie z.B. Senkung des peripheren arteriellen, des koronaren und des pulmonalen Gefäßwiderstandes, Senkung des pulmonalen Blutdrucks, Senkung des systemischen Blutdrucks ohne zugleich Schlagvolumen und koronare Durchblutung zu senken, Förderung der Nierendurchblutung und der Durchblutung anderer peripherer Organe, Erhöhung der cerebralen Durchblutung, Inhibierung der Thrombozytenaktivierung und Auflösung von Thromben, Inhibierung der Bronchokonstriktion, Inhibierung der Magensäuresekretion, Zytoprotektion des Herzens, der Magen- und Darmschleimhaut, der Leber, Zytoprotektion im Pankreas und in der Niere sowie antiallergische Eigenschaften. Daher sind die neuen TXA₂-Antagonisten prinzipiell geeignet zur Behandlung des Schlaganfalles, der Prophylaxe und Therapie koronarer Herzerkrankungen, zum Beispiel der Koronarthrombose, zur Behandlung des Herzinfarktes, peripherer Arterienerkrankungen, zur Prophylaxe und Therapie bei anderen thromboembolischen Erkrankungen und bei Arteriosklerose, bei ischämischen Attacken des ZNS-Systems und anderer Durchblutungsstörungen des Gehirns, zur Behandlung der Hypertonie und zur Behandlung von Krankheiten, die mit einer Erhöhung des pulmonalen Gefäßwiderstandes einhergehen wie z.B. der pulmonalen Hypertonie und zur Therapie des Schocks und des Asthmas. Sie können ferner eingesetzt werden, zur Inhibierung von Geburtswehen und zur Behandlung von Schwangerschaftstoxikosen.
Die neuen TXA₂-Antagonisten können außerdem Anwendung finden zur Verbesserung der Organfunktion nach Transplantation, zum Beispiel bei der Nierentransplantation, zur Verhinderung von Abstoßungsreaktionen, an Stelle von Heparin oder als Adjuvans bei der Dialyse oder Hämofiltration und bei der Konservierung von Blutplasmakonserven, zum Beispiel von Blutplättchenkonserven.
Die neuen TXA₂-Antagonisten besitzen eine antimetastatische Wirkung und antiproliferative Eigenschaften. Sie sind prinzipiell geeignet zur Behandlung Hormonabhängiger Neoplasien.
Die neuen TXA₂-Antagonisten können in Kombination, zum Beispiel mit Carbacyclinen, Prostacyclin und seinen Analoga, 7-Oxo-prostacyclinen, Prostaglandinen und deren Derivate und 6-Oxo-PGE₁- und 6-Oxo-9-Fluor-Prastaglandin-Derivaten, mit TXA₂-Synthetase-Inhibitoren, mit Phosphodiesterase-Inhibitoren, mit Antagonisten und Rezeptorantagonisten verschiedener Throbozytenstimulatoren (z.B. ADP, Thrombin, Collagen, PAF, Adrenalin, Serotonin, Fibrinogen), mit Calciumantagonisten, mit Fibrinolytika und Thrombolytika, z.B. t-PA, mit Heparin und anderen Antikoagulanzien, mit Cyclooxygenasehemmern, z.B. Acetylsalicylsäure, mit Hemmstoffen der Lipoxygenasen sowie Antagonisten von Lipoxygenaseprodukten, mit Vasodilatatoren wie z.B. Nitroverbindungen, mit Antihypertensiva wie z.B. β-Blockern oder mit Diuretika Verwendung finden.
Die Dosis der Verbindungen ist 0,1-500 mg/Tag, auch in mehreren Teildosierungen, wenn sie am menschlichen Patienten verabreicht werden. Die Einheitsdosis für den pharmazeutisch akzeptablen Träger beträgt 0,1-100 mg. Für die parenterale Verabreichung werden sterile, injizierbare wässrige oder ölige Lösungen benutzt. Für die orale Applikation sind beispielsweise Tabletten, Dragees oder Kapseln geeignet.
Die Erfindung betrifft damit auch Arzneimittel auf Basis der Verbindungen der allgemeinen Formel I und üblicher Hilfs- und Trägerstoffe.
Die erfindungsgemäßen Wirkstoffe sollen in Verbindung mit den in der Galenik bekannten und üblichen Hilfsstoffen z.B. zur Herstellung von Blutdrucksenkern dienen.
Der Einheitsdosisberich für die Ampulle ist 0,1-100 mg, für die Tablette 0,1-100 mg.

### Beispiel 1:

### 5-{(E)-(1S,5S,6S,7R)-7-Hydroxy-6-[(E/Z)-3-phenylureidoiminomethyl]-bicyclo[3.3.0]oct-3-yliden}-pentansäure:

Die farblose Lösung von 64 mg (160 µmol) der nach Beispiel 1a dargestellten Verbindung in 4,5 ml Methanol versetzt man mit 1,8 ml einer 10%-igen wässrigen Kaliumhydroxidlösung und läßt 16 Stunden bei 23°C rühren. Durch Zugabe von gesättigter Zitronensäurelösung stellt man einen pH von 3-4 ein, sättigt mit Natriumchlorid und extrahiert mehrfach mit insgesamt 20 ml Dichlormethan. Den nach Trocknung über Magnesiumsulfat, Filtration und Lösungsmittelabzug erhaltenen Rückstand reinigt man durch Chromatographie an vier analytischen Dünnschichtplatten. Als Laufmittel dient ein Gemisch aus Dichlormethan und Methanol, als Elutionsmittel Trichlormethan und Ethanol. Isoliert werden 35 mg (91 µmol, 57%) der Titelverbindung als farbloses Öl.
IR (Film): 3600-2500, 3380, 3340, 3090, 2940, 1710, 1670, 1595, 1540, 1450, 750 und 690 cm⁻¹.

### Beispiel 1a:

### 5-{(E)-(1S,5S,6S,7R)-7-Hydroxy-6-[(E/Z)-3-phenylureidoiminomethyl]-bicyclo[3.3.0]oct-3-yliden}-pentansäuremethylester:

98 mg (202 µmol) der nach Beispiel 1b dargestellten Verbindung löst man in 2,5 ml wasserfreiem Ethanol, versetzt mit 25 mg Pyridinium-p-toluolsulfonat und erwärmt 3 Stunden unter einer Atmosphäre aus trockenem Argon auf 55°C. Nach dem Erkalten versetzt man mit einer 50%-igen Natriumchloridlösung und extrahiert mehrfach mit Dichlormethan. Den nach Trocknung über Magnesiumsulfat, Filtration und Lösungsmittelabzug erhaltenen Rückstand reinigt man durch Chromatographie an vier analytischen Dünnschichtplatten. Als Laufmittel dient ein Gemisch aus n-Hexan und Ethylacetat, als Elutionsmittel Ethylacetat. Isoliert werden 64 mg (160 µmol, 79%) der Titelverbindung als farbloses Öl.
IR (Film): 3500-3200, 3380, 3340, 3080, 2940, 1735, 1670, 1595, 1535, 1450, 750 und 690 cm⁻¹.

### Beispiel 1b:

### 5-{(E)-(1S,5S,6S,7R)-7-(Tetrahydropyran-2-yloxy)-6-[(E/Z)-3-phenylureidoiminomethyl]-bicyclo[3.3.0]oct-3-yliden}-pentansäuremethylester:

Die Lösung von 88 mg (236 µmol) des nach Beispiel 1c dargestellten Aldehyds in 2 ml wasserfreiem Ethanol versetzt man mit 48 mg 4-Phenylsemicarbazid, 2 Tropfen Pyridin und erwärmt 3 Stunden unter einer Atmosphäre aus trockenem Argon auf 55°C. Die Aufarbeitung erfolgt wie in Beispiel 1a beschrieben. Nach Chromatographie an ca. 30 ml feinem Kieselgel unter Verwendung eines Gradientensystems aus n-Hexan und Ethylacetat isoliert man 98 mg (202 µmol, 86%) der Titelverbindung als farbloses Öl.
IR (Film): 3400-3300, 2950, 2920, 2850, 1730, 1680, 1590, 1530, 1460, 1445 und 740 cm⁻¹.

### Beispiel 1c:

### 5-{(E)-(1S,5S,6R,7R)-7-(Tetrahydropyran-2-yloxy)-6-formyl-bicyclo[3.3.0]oct-3-yliden}-pentansäuremethylester:

Zu der Lösung von 179 µl frisch destilliertem Oxalylchlorid in 3,6 ml wasserfreiem Dichlormethan tropft man unter einer Atmosphäre aus trockenem Argon die Lösung von 326 µl wasserfreiem Dimethylsulfoxid in 1,45 ml Dichlormethan bei -60°C zu, rührt noch 15 Minuten nach und versetzt mit der Lösung aus 500 mg (1,42 mmol) des nach Beispiel 1d dargestellten Alkohols in 2,88 ml Dichlormethan. Man läßt 2,5 Stunden reagieren, quencht durch Zugabe von 559 µl Triethylamin, läßt auf 23°C erwärmen, verdünnt mit Wasser, trennt die organische Phase ab und extrahiert die wässrige Phase mehrfach mit Dichlormethan. Die vereinigten organischen Extrakte trocknet man über Magnesiumsulfat und isoliert nach Filtration und Lösungsmittelabzug 500 mg (1,42 mmol, 100%) der Titelverbindung als farbloses Öl, das man ohne Reinigung weiter umsetzt.

### Beispiel 1d:

### 5-{(E)-(1S,5S,6S,7R)-7-(Tetrahydropyran-2-yloxy)-6-hydroxymethyl-bicyclo[3.3.0]oct-3-yliden}-pentansäuremethylester:

9,18 g (15,5 mmol) des nach Beispiel 1e dargestellten Esters löst man in 180 ml wasserfreiem Tetrahydrofuran, versetzt mit 37,3 ml einer 1M Lösung von Tetraburylammoniumfluorid in Tetrahydrofuran und rührt 12 Stunden bei 23°C unter einer Atmosphäre aus trockenem Argon. Man gießt in Eiswasser, extrahiert mehrfach mit Dichlormethan, trocknet die vereinigten organischen Extrakte über Magnesiumsulfat und isoliert nach Filtration und Lösungsmittelabzug 11,3 g Rohöl, das man durch Chromatographie an ca. 400 ml grobem Kieselgel unter Verwendung eines Gradientensystems aus n-Hexan und Ethylacetat auftrennt. Isoliert werden 3,94 g (11,2 mmol, 72%) der Titelverbindung als farbloses Öl.
IR (Film): 3600-3200, 2940, 2860, 1735, 1435, 1350, 1200, 1160, 1130, 1075, 1020, 975, 865 und 810 cm⁻¹.

### Beispiel 1e:

### 5-{(E)-(1S,5S,6S,7R)-7-(Tetrahydropyran-2-yloxy)-6-tert.-buryl-diphenylsilyloxymethyl-bicyclo[3.3.0]oct-3-yliden}-pentansäuremethylester:

Die Lösung von 11,67 g (20,2 mmol) der nach Beispiel 1f dargestellten polaren Säure A in 100 ml Dichlormethan kühlt man auf 3°C und verestert durch Zugabe einer etherischen Lösung von Diazomethan. Den nach Lösungsmittelabzug erhaltenen Rückstand reinigt man durch Chromatographie an ca. 500 ml feinem Kieselgel unter Verwendung eines 8:2-Gemisches aus n-Hexan und Ethylacetat. Isoliert werden 9,18 g (15,5 mmol, 77%) der Titelverbindung als farbloses Öl.
IR (Film): 3070, 3040, 2950, 2930, 2850, 1735, 1450, 1425, 1355, 1200, 1130, 1110, 1075, 1020, 865, 820, 740 und 700 cm⁻¹.

### Beispiel 1f:

### 5-{(E)-(1S,5S,6S,7R)-7-(Tetrahydropyran-2-yloxy)-6-tert.-butyl-diphenylsilyloxymethyl-bicyclo[3.3.0]oct-3-yliden}-pentansäure (A) und 5-[(Z)-(1S,5S,6S,7R)-7-(Tetrahydropyran-2-yloxy)-6-tert.butyl-phenylsilyloxymethyl-bicyclo[3.3.0]oct-3-yliden}-pentansäure (B):

Die Emulsion aus 117,5 g Carboxyburyltriphenylphosphoniumbromid in einem Gemisch aus wasserfreiem Dimethylsulfoxid und Tetrahydrofuran kühlt man unter einer Atmosphäre aus trockenem Argon auf 3°C und versetzt innerhalb 0,5 Stunden portionsweise mit insgesamt 59,4 g Kalium-t-butanolat. Man rührt noch 0,5 Stunden nach und tropft zu der klaren roten Lösung innerhalb 10 Minuten die Lösung von 21,75 g (44,1 mmol) des nach Beispiel 1g dargestellten Ketons in 40 ml wasserfreiem Tetrahydrofuran. Man erwärmt 1,5 Stunden auf 35°C, gießt auf 1l Eiswasser, stellt durch Zugabe einer gesättigten Zitronensäurelösung einen pH-Wert von 4 ein und extrahiert mehrfach mit Diethylether. Die vereinigten organischen Extrakte wäscht man mit gesättigter Natriumchloridlösung, trocknet über Magnesiumsulfat und reinigt den nach Filtration und Lösungsmittelabzug erhaltenen Rückstand durch wiederholte Chromatographie an mittelfeinem Kieselgel unter Verwendung eines Gradientensystems aus n-Hexan und Ethylacetat. Isoliert werden 11,67 g (20,2 mmol, 46%) der polaren Titelverbindung A, sowie 7,44 g (12,9 mmol, 29%), der unpolaren Titelverbindung B, jeweils als farbloses Öl.
IR (Film) von A und B: 3600-2500, 3070, 3050, 3010, 2940, 2850, 1705, 1425, 1355, 1200, 1125, 1110, 1075, 1020, 865, 815, 740 und 695 cm⁻¹.

### Beispiel 1g:

### (1S,2S,3R,5R)-3-(Tetrahydropyran-2-yloxy)-2-tert.-butyl-diphenylsilyloxymethyl-bicyclo-[3.3.0]octan-7-on:

Die Lösung von 25,77 g (63 mmol) des nach Beispiel 1h dargestellten Alkohols in 250 ml wasserfreiem Dichlormethan versetzt man mit 6,71 ml Dihydropyran, 71 mg p-Toluolsulfonsäurechlorid und läßt 1,5 Stunden bei 23°C unter einer Atmosphäre aus trockenem Argon reagieren. Man wäscht mit einer gesättigten Natriumhydiogencarbonatlösung und gesättigter Natriumchloridlösung neutral, trocknet über Magnesiumsulfat und reinigt den nach Filtration und Lösungsmittelabzug erhaltenen Rückstand durch Chromatographie an ca. 1l feinem Kieselgel unter Verwendung eines Gradientensystems aus n-Hexan und Ethylacetat. Isoliert werden 25,75 g (52,2 mmol, 83%) der Titelverbindung als farbloses Öl.
IR (Film): 3060, 3020, 2930, 2850, 1735, 1590, 1465, 1425, 1200, 1110, 1020, 970, 865, 820, 740 und 700 cm⁻¹.

### Beispiel 1h:

### (1S,2S,3R,5R)-3-hydroxy-2-tert.-butyl-diphenylsilyloxymethyl-7,7-(2,2-dimethyl-trimethylendioxy)-bicyclo[3.3.0]octan:

43,23 g (74,7 mmol) des nach Beispiel 1i dargestellten Ketals versetzt man mit 1l eines 65:35:10-Gemisches aus Essigsäure, Wasser, Tetrahydrofuran und rührt 18 Stunden bei 23°C. Man engt ein und entfernt restliche Essigsäure durch wiederholte Zugabe von Toluol azeotrop. Den Rückstand reinigt man durch Chromatographie an 900 ml feinem Kieselgel unter Verwendung eines Gradientensystems aus n-Hexan und Ethylacetat. Isoliert werden 22,65 g (55,4 mmol, 86%) kristalline Titelverbindung neben 4,16 g (8,44 mmol, 13%) der Titelverbindung aus Beispiel 1g.
IR (CHCl₃): 3600-3200, 3070, 2940, 2850, 1730, 1590, 1465, 1425, 1400, 1255, 1110, 1040, 1005, 900, 860, 820 und 700 cm⁻¹.

### Beispiel 1i:

### (1S,2S,3R,5R)-3-(Tetrahydropyran-2-yloxy)-2-tert.-butyl-diphenylsilyloxymethyl-7,7-(2,2-dimethyl-trimethylendioxy)-bicyclo[3.3.0]octan:

22,0 g (64,6 mmol) des nach Beispiel 1j dargestellten Alkohols löst man in 200 ml wasserfreiem Dimethylformamid, versetzt mit 10 g Imidazol, 21 ml tert.-Butyldiphenylchlorsilan und rührt 14 Stunden bei 23°C unter einer Atmosphäre aus trockenem Argon. Man gießt in 500 ml Eiswasser, das mit 300 ml Diethylether überschichtet ist, trennt die organische Phase ab, extrahiert die wässrige Phase mehrfach mit insgesamt 1,5 l Diethylether, wäscht die vereinigten organischen Extrakte mit Wasser und gesättigter Natriumchloridlösung, trocknet über Magnesiumsulfat und reinigt den nach Filtration und Lösungsmittelabzug erhaltenen Rückstand durch Chromatographie an ca. 500 ml feinem Kieselgel unter Verwendung eines Gradientensystems aus n-Hexan und Ethylacetat. Isoliert werden 36,6 g (63,2 mmol, 98%) der Titelverbindung als erstarrtes Öl.
IR (CHCl₃): 3060, 3000, 2950, 2850, 1590, 1470, 1425, 1390, 1350, 1320, 1255, 1105, 1075, 1020, 910, 865, 820 und 700 cm⁻¹.

### Beispiel 1j:

### (1S,2S,3R,5R)-3-(Tetrahydropyran-2-yloxy)-7,7-(2,2-dimethyl-trimethylendioxy)-bicyclo[3.3.0]octan-2-methanol:

24,3 g (66 mmol) des nach Beispiel 1k dargestellten Esters löst man in 200 ml wasserfreiem Toluol, kühlt unter einer Atmosphäre aus trockenem Argon auf -20°C und tropft innerhalb 30 Minuten 55 ml einer 1,1M Lösung von Diisobutylaluminiumhydrid in Toluol so zu, daß die Innentemperatur -5°C nicht überschritten wird. Man läßt noch 30 Minuten bei -5°C reagieren, versetzt mit 12 ml Isopropanol und 11 ml Wasser, läßt auf 23°C erwärmen und rührt so lange, bis ein feinkörniger Niederschlag entsteht. Nach Filtration und Lösungsmittelabzug isoliert man 22,06 g (64,8 mmol, 98%) der Titelverbindung als kristallinen Feststoff.
IR (CHCl₃): 3600-3300, 2950, 2860, 1465, 1350, 1325, 1255, 1155, 1115, 1070, 1020, 975, 905, 865 und 810 cm⁻¹.

### Beispiel 1k:

### (1S,2S,3R,5R)-3-(Tetrahydropyran-2-yloxy)-7,7-(2,2-dimethyl-trimethylendioxy)-bicyclo[3.3.0]octan-2-carbonsäuremethylester:

20,0 g (70,3 mmol)(1S,2S,3R,5R)-3-hydroxy-7,7-(2,2-dimethyl-trimethylendioxy)-bicyclo[3.3.0]octan-2-carbonsäuremethylester setzt man in Analogie zu Beispiel 1g um und isoliert nach Aufarbeitung und Reinigung 24,8 g (67,3 mmol, 96%) der Titelverbindung als kristallinen Feststoff.
IR (CHCl₃): 2950, 2860, 1725, 1435, 1255, 1165, 1115, 1075, 1030, 905, 865 und 815 cm⁻¹.

### Beispiel 2:

### 5-{(Z)-(1S,5S,6S,7R)-7-Hydroxy-6-[(E/Z)-3-phenylureidoiminomethyl]-bicyclo[3.3.0]oct-3-yliden}-pentansäure:

87 mg (218 µmol) des nach Beispiel 2a dargestellten Esters verseift man in Analogie zu Beispiel 1 und isoliert nach Aufarbeitung und Reinigung 36 mg (93 µmol, 43%) der Titelverbindung als farbloses Öl.
IR (Film): 3600-2500, 3370, 3220, 3100, 2950, 2930, 2850, 1710, 1680, 1595, 1540, 1450, 1235, 910, 755, 735 und 690 cm⁻¹.

### Beispiel 2a:

### 5-{(Z)-(1S,5S,6S,7R)-7-Hydroxy-6-[(E/Z)-3-phenylureidoiminomethyl]-bicyclo[3.3.0]oct-3-yliden}-pentansäuremethylester:

113 mg (233 µmol) der nach Beispiel 2b dargestellten Verbindung setzt man in Analogie zu Beispiel 1a um und isoliert nach Aufarbeitung und Reinigung 87 mg (218 µmol, 93%) der Titelverbindung als farbloses Öl.
IR (Film): 3500-3200, 3380, 3100, 2950, 2930, 2850, 1735, 1675, 1595, 1540, 1445, 1235, 910, 755, 735 und 690 cm⁻¹.

### Beispiel 2b:

### 5-{(Z)-(1S,5S,6S,7R)-7-(Tetrahydropyran-2-yloxy)-6-[(E/Z)-3-phenylureidoiminomethyl]-bicyclo[3.3.0]oct-3-yliden}-pentansäuremethylester:

87 mg (248 µmol) des nach Beispiel 2c dargestellten Aldehyds setzt man in Analogie zu Beispiel 1b unter Verwendung von 4-Phenylsemicarbazid um und isoliert nach Aufarbeitung und Reinigung 113 mg (233 µmol, 94%) der Titelverbindung als farbloses Öl.
IR (Film): 3400-3200, 2940, 2850, 1735, 1685, 1590, 1330, 1120, 1075, 1025, 970, 865, 815, 750 und 690 cm⁻¹.

### Beispiel 2c:

### 5-{(Z)-(1S,5S,6R,7R)-7-(Tetrahydropyran-2-yloxy)-6-formyl-bicyclo[3.3.0]oct-3-yliden}-pentansäuremethylester:

500 mg (1,42 mmol) des nach Beispiel 2d dargestellten Alkohols oxidiert man in Analogie zu Beispiel 1c und isoliert nach Aufarbeitung 501 mg (1,42 mmol, 100%) der Titelverbindung als farbloses Öl.

### Beispiel 2d:

### 5-{(Z)-(1S,5S,6S,7R)-7-(Tetrahydropyran-2-yloxy)-6-hydroxymethyl-bicyclo[3.3.0]oct-3-yliden}-pentansäuremethylester:

7,16 g (12,1 mmol) der nach Beispiel 2e dargestellten Verbindung setzt man in Analogie zu Beispiel 1d um und isoliert nach Aufarbeitung und Reinigung 3,35 g (9,5 mmol, 78%) der Titelverbindung als farbloses Öl.
IR (Film): 3600-3200, 2940, 2870, 1735, 1435, 1350, 1245, 1200, 1160, 1135, 1075, 1020, 975, 910, 865 und 810 cm⁻¹.

### Beispiel 2e:

### 5-{(Z)-(1S,5S,6S,7R)-7-(Tetrahydropyran-2-yloxy)-6-tert.-butyl-diphenylsilyloxymethyl-bicyclo[3.3.0]oct-3-yliden}-pentansäuremethylester:

7,44 g (12,9 mmol) der nach Beispiel 1f dargestellten unpolareren Säure B verestert man in Analogie zu Beispiel 1e und isoliert nach Aufarbeitung und Reinigung 7,16 g (12,1 mmol, 94%) der Titelverbindung als farbloses Öl.
IR (Film): 3070, 3050, 2940, 2850, 1735, 1450, 1425, 1355, 1200, 1125, 1110, 1080, 1020, 865, 815, 740 und 700 cm⁻¹.

### Beispiel 3:

### 5-{(E)-(1S,5S,6S,7R)-7-Hydroxy-6-[(E/Z)-cyclohexylmethoxylminomethyl]-bicyclo[3.3.0]oct-3-yliden}-pentansäure:

46 mg (122 µmol) des nach Beispiel 3a dargestellten Esters verseift man in Analogie zu Beispiel 1 und isoliert nach Aufarbeitung und Reinigung 31 mg (86 µmol, 70%) der Titelverbindung als farbloses Öl.
IR (Film): 3600-2500, 2920, 2850, 1710, 1450, 1385, 1080, 1040 und 1025 cm⁻¹.

### Beispiel 3a:

### 5-{(E)-(1S,5S,6S,7R)-7-Hydroxy-6-[(E/Z)-cyclohexylmethoxyiminomethyl]-bicyclo[3.3.0]oct-3-yliden}-pentansäuremethylester:

70 mg (152 µmol) der nach Beispiel 3b dargestellten Verbindung setzt man in Analogie zu Beispiel 1a um und isoliert nach Aufarbeitung und Reinigung 46 mg (122 µmol, 80%) der Titelverbindung als farbloses Öl.
IR (Film): 3500-3200, 2930, 2850, 1730, 1450, 1385, 1080, 1040 und 1025 cm⁻¹.

### Beispiel 3b:

### 5-{(E)-(1S,5S,6S,7R)-7-(Tetrahydropyran-2-yloxy)-6-[(E/Z)-cyclohexylmethoxyiminomethyl]-bicyclo[3.3.0]oct-3-yliden}-pentansäuremethylester:

82 mg (234 µmol) des nach Beispiel 1c dargestellten Aldehyds setzt man in Analogie zu Beispiel 1b unter Verwendung von Cyclohexylmethoxyamin um und isoliert nach Aufarbeitung und Reinigung 70 mg (152 µmol, 65%) der Titelverbindung als farbloses Öl.
IR (Film): 2940, 2850, 1735, 1450, 1380, 1080, 1040, 1025, 865 und 814 cm⁻¹.

### Beispiel 4:

### 5-{(Z)-(1S,5S,6S,7R)-7-Hydroxy-6-[E/Z)-cyclohexylmethoxyiminomethyl]-bicyclo[3.3.0]oct-3-yliden}-pentansäure:

70 mg (186 µmol) des nach Beispiel 4a dargestellten Esters verseift man in Analogie zu Beispiel 1 und isoliert nach Aufarbeitung und Reinigung 56 mg (154 µmol, 83%) der Titelverbindung als farbloses Öl.
IR (Film): 3600-2500, 2930, 2850, 1710, 1450, 1080, 1035 und 1025 cm⁻¹.

### Beispiel 4a:

### 5-{(Z)-(1S,5S,6S,7R)-7-Hydroxy-6-[E/Z)-cyclohexylmethoxyiminomethyl]-bicyclo[3.3.0]oct-3-yliden}-pentansäuremethylester:

96 mg (214 µmol) der nach Beispiel 4b dargestellten Verbindung setzt man in Analogie zu Beispiel 1a um und isoliert nach Aufarbeitung und Reinigung 70 mg (186 µmol, 87%) der Titelverbindung als farbloses Öl.
IR (Film): 3500-3200, 2930, 2850, 1735, 1450, 1080, 1035 und 1025 cm⁻¹.

### Beispiel 4b:

### 5-{(Z)-(1S,5S,6S,7R)-7-(Tetrahydropyran-2-yloxy)-6-[(E/Z)-cyclohexylmethoxyiminomethyl]-bicyclo[3.3.0]oct-3-yliden}-pentansäuremethylester:

83 mg (236 µmol) des nach Beispiel 2c dargestellten Aldehyds setzt man in Analogie zu Beispiel 1b unter Verwendung von Cyclohexylmethoxyamin um und isoliert nach Aufarbeitung und Reinigung 96 mg (214 µmol, 91%) der Titelverbindung als farbloses Öl. IR (Film): 2940, 2850, 1735, 1445, 1080, 1035, 1025, 860 und 810 cm⁻¹.

### Beispiel 5:

### 5-{(E)-(1S,5S,6S,7R)-7-Hydroxy-6-[(E/Z)-4-trifluormethylbenzyloxyiminomethyl]-bicyclo [3.3.0]oct-3-yliden}-pentansäure:

77 mg (175 µmol) des nach Beispiel 5a dargestellten Esters verseift man in Analogie zu Beispiel 1 und isoliert nach Aufarbeitung und Reinigung 67 mg (157µmol, 90%) der Titelverbindung als farbloses Öl.
IR (Film): 3600-2500, 2930, 2870, 1710, 1620, 1325, 1165, 1125, 1065 und 1015 cm⁻¹.

### Beispiel 5a:

### 5-{(E)-(1S,5S,6S,7R)-7-Hydroxy-6-[E/Z)-4-trifluormethylbenzyloxyiminomethyl]-bicyclo [3.3.0]oct-3-yliden}-pentansäuremethylester:

106 mg (202 µmol) der nach Beispiel 5b dargestellten Verbindung setzt man in Analogie zu Beispiel 1a um und isoliert nach Aufarbeitung und Reinigung 77 mg (175 µmol, 87%) der Titelverbindung als farbloses Öl.
IR (Film): 3500-3200, 2930, 2860, 1735, 1620, 1325, 1165, 1125, 1065 und 1015 cm⁻¹.

### Beispiel 5b:

### 5-{(E)-(1S,5S,6S,7R)-7-Tetrahydropyran-2-yloxy)-6-[(E/Z)-4-trifluormethylbenzyloxy-iminomethyl]-bicyclo[3.3.0]oct-3-yliden}-pentansäuremethylester:

83 mg (236 µmol) des nach Beispiel 1c dargestellten Aldehyds setzt man in Analogie zu Beispiel 1b unter Verwendung von 4-Trifluormethylphenylmethoxyamin um und isoliert nach Aufarbeitung und Reinigung 106 mg (202 µmol, 86%) der Titelverbindung als farbloses Öl.
IR (Film): 2930, 2860, 1735, 1620, 1325, 1165, 1125, 1065, 1015, 865 und 820 cm⁻¹.

### Beispiel 6:

### 5-{(Z)-(1S,5S,6S,7R)-7-Hydroxy-6-[(E/Z)-4-trifluormethylbenzyloxyiminomethyl]-bicyclo [3.3.0]oct-3-yliden}-pentansäure:

55 mg (126 µmol) des nach Beispiel 6a dargestellten Esters verseift man in Analogie zu Beispiel 1 und isoliert nach Aufarbeitung und Reinigung 51 mg (97 µmol, 77%) der Titelverbindung als farbloses Öl.
IR (Film): 3600-2400, 2940, 2870, 1710, 1620, 1420, 1325, 1165, 1125, 1065 und 1020 cm⁻¹.

### Beispiel 6a:

### 5-{(Z)-(1S,5S,6S,7R)-7-Hydroxy-6-[(E/Z)-4-trifluormethylbenzyloxyiminomethyl]-bicyclo [3.3.0]oct-3-yliden}-pentansäuremethylester:

96 mg (183 µmol) der nach Beispiel 6b dargestellten Verbindung setzt man in Analogie zu Beispiel 1a um und isoliert nach Aufarbeitung und Reinigung 55 mg (126 µmol, 69%) der Titelverbindung als farbloses Öl.
IR (Film): 3500-3200, 2940, 2870, 1730, 1620, 1420, 1325, 1165, 1125, 1065 und 1020 cm⁻¹.

### Beispiel 6b:

### 5-{(Z)-(1S,5S,6S,7R)-7-(Tetrahydropyran-2-yloxy)-6-[(E/Z)-4-trifluormethylbenzyloxyiminomethyl]-bicyclo[3.3.0]oct-3-yliden}-pentansäuremethylester:

83 mg (236 µmol) des nach Beispiel 2c dargestellten Aldehyds setzt man in Analogie zu Beispiel 1b unter Verwendung von 4-Trifluormethylphenylmethoxyamin um und isoliert nach Aufarbeitung und Reinigung 96 mg (183 µmol, 78%) der Titelverbindung als farbloses Öl.
IR (Film): 2940, 2850, 1735, 1435, 1325, 1160, 1120, 1065, 1020, 865 und 815 cm⁻¹.

### Beispiel 7:

### 5-{(E)-(1S,5S,6S,7R)-7-Hydroxy-6-[(E/Z)-4-fluorbenzyloxyiminomethyl]-bicyclo[3.3.0]oct-3-yliden}-pentansäure:

69 mg (177 µmol) des nach Beispiel 7a dargestellten Esters verseift man in Analogie zu Beispiel 1 und isoliert nach Aufarbeitung und Reinigung 62 mg (165 µmol, 93%) der Titelverbindung als farbloses Öl.
IR (Film): 3600-2500, 3050, 2930, 2870, 1710, 1605, 1510, 1225, 1155, 1080 und 1025 cm⁻¹.

### Beispiel 7a:

### 5-{(E)-(1S,5S,6S,7R)-7-Hydroxy-6-[(E/Z)-4-fluorbenzyloxyiminomethyl]-bicyclo[3.3.0]oct-3-yliden}-pentansäuremethylester:

90 mg (190 µmol) der nach Beispiel 7b dargestellten Verbindung setzt man in Analogie zu Beispiel 1a um und isoliert nach Aufarbeitung und Reinigung 69 mg (177 µmol, 93%) der Titelverbindung als farbloses Öl.
IR (Film): 3500-3200, 3050, 2930, 2870, 1730, 1605, 1510, 1220, 1155, 1080 und 1025 cm⁻¹.

### Beispiel 7b:

### 5-{(E)-(1S,5S,6S,7R)-7-(Tetrahydropyran-2-yloxy)-6-[(E/Z)-4-fluorbenzyloxyiminomethyl] -bicyclo[3.3.0]oct-3-yliden}-pentansäuremethylester:

83 mg (236 µmol) des nach Beispiel 1c dargestellten Aldehyds setzt man in Analogie zu Beispiel 1b unter Verwendung von 4-Fluorphenylmethoxyamin um und isoliert nach Aufarbeitung und Reinigung 90 mg (190 µmol, 81%) der Titelverbindung als farbloses Öl.
IR (Film): 3050, 2940, 2870, 1735, 1605, 1510, 1220, 1155, 1080, 1025, 860 und 810 cm⁻¹.

### Beispiel 8:

### 5-{(Z)-(1S,5S,6S,7R)-7-Hydroxy-6-[(E/Z)-4-fluorbenzyloxymininomethyl]-bicyclo[3.3.0]-oct-3-yliden}-pentansäure:

63 mg (167 µmol) des nach Beispiel 8a dargestellten Esters verseift man in Analogie zu Beispiel 1 und isoliert nach Aufarbeitung und Reinigung 52 mg (139 µmol, 83%) der Titelverbindung als farbloses Öl.
IR (Film): 3600-2400, 3050, 2930, 2870, 1710, 1600, 1510, 1225, 1080, 1015 und 830 cm⁻¹.

### Beispiel 8a:

### 5-{(Z)-(1S,5S,6S,7R)-7-Hydroxy-6-[(E/Z)-4-fluorbenzyloxyiminomethyl]-bicyclo[3.3.0]oct-3-yliden}-pentansäuremethylester:

85 mg (185 µmol) der nach Beispiel 8b dargestellten Verbindung setzt man in Analogie zu Beispiel 1a um und isoliert nach Aufarbeitung und Reinigung 63 mg (167 µmol, 90%) der Titelverbindung als farbloses Öl.
IR (Film): 3500-3200, 3050, 2940, 2870, 1735, 1600, 1510, 1225, 1080, 1015, und 830 cm⁻¹.

### Beispiel 8b:

### 5-{(Z)-(1S,5S,6S,7R)-7-(Tetrahydropyran-2-yloxy)-6[(E/Z)-4-fluorbenzyloxriminomethyl] -bicyclo[3.3.0]oct-3-yliden}-pentansäuremethylester:

83 mg (236 µmol) des nach Beispiel 2c dargestellten Aldehyds setzt man in Analogie zu Beispiel 1b unter Verwendung von 4-Fluorphenylmethoxyamin um und isoliert nach Aufarbeitung und Reinigung 85 mg (185 µmol, 78%) der Titelverbindung als farbloses Öl.
IR (Film): 3040, 2940, 2860, 1735, 1600, 1510, 1435, 1355, 1220, 1155, 1120, 1075, 1030, 1020, 870 und 820 cm⁻¹.

### Beispiel 9:

### 5-{(E)-(1S,5S,6S,7R)-7-Hydroxy-6-[(E/Z)-benzyloxyiminomethyl]-bicyclo[3.3.0]oct-3-yliden}-pentansäure:

52 mg (140 µmol) des nach Beispiel 9a dargestellten Esters verseift man in Analogie zu Beispiel 1 und isoliert nach Aufarbeitung und Reinigung 49 mg (137 µmol, 98%) der Titelverbindung als farbloses Öl.
IR (Film): 3600-2500, 3030, 2930, 2870, 1705, 1455, 1385, 1240, 1080, 1020 und 700 cm⁻¹.

### Beispiel 9a:

### 5-{(E)-(1S,5S,6S,7R)-7-Hydroxy-6-[(E/Z)-benzyloxyiminomethyl]-bicyclo[3.3.0]oct-3-yliden}-pentansäuremethylester:

79 mg (173 µmol) der nach Beispiel 9b dargestellten Verbindung setzt man in Analogie zu Beispiel 1a um und isoliert nach Aufarbeitung und Reinigung 52 mg (140 µmol, 81%) der Titelverbindung als farbloses Öl.
IR (Film): 3500-3200, 3030, 2930, 2870, 1730, 1455, 1380, 1240, 1080, 1020 und 700 cm⁻¹.

### Beispiel 9b:

### 5-{(E)-(1S,5S,6S,7R)-7-(Tetrahydropyran-2-yloxy)-6-[(E/Z)-benzyloxyiminomethyl]-bicyclo[3.3.0]oct-3-yliden}-pentansäuremethylester:

83 mg (236 µmol) des nach Beispiel 1c dargestellten Aldehyds setzt man in Analogie zu Beispiel 1b unter Verwendung von Phenylmethoxyamin um und isoliert nach Aufarbeitung und Reinigung 79 mg (173 µmol, 73%) der Titelverbindung als farbloses Öl. IR (Film): 3000, 2930, 2870, 1735, 1455, 1380, 1240, 1080, 1020, 865, 815 und 700 cm⁻¹.

### Beispiel 10:

### 5-{(Z)-(1S,5S,6S,7R)-7-Hydroxy-6-((E/Z)-benzyloxyiminomethyl]-bicyclo[3.3.0]oct-3-yliden}-pentansäure:

60 mg (167 µmol) des nach Beispiel 10a dargestellten Esters verseift man in Analogie zu Beispiel 1 und isoliert nach Aufarbeitung und Reinigung 54 mg (151 µmol, 90%) der Titelverbindung als farbloses Öl.
IR (Film): 3600-2400, 3060, 3030, 2930, 2870, 1705, 1505, 1245, 1080, 1015, 920 und 695 cm⁻¹.

### Beispiel 10a:

### 5-{(Z)-(1S,5S,6S,7R)-7-Hydroxy-6-[(E/Z)-benzyloxyiminomethyl]-bicyclo[3.3.0]oct-3-yliden}-pentansäuremethylester:

79 mg (178 µmol) der nach Beispiel 10b dargestellten Verbindung setzt man in Analogie zu Beispiel 1a um und isoliert nach Aufarbeitung und Reinigung 60 mg (167 µmol, 94%) der Titelverbindung als farbloses Öl.
IR (Film): 3500-3200, 3050, 3030, 2930, 2870, 1735, 1505, 1240, 1080, 1015, 920 und 695 cm⁻¹.

### Beispiel 10b:

### 5-{(Z)-(1S,5S,6S,7R)-7-(Tetrahydropyran-2-yloxy)-6-[(E/Z)-benzyloxyiminomethyl]-bicyclo[3.3.0]oct-3-yliden}-pentansäuremethylester:

83 mg (236 µmol) des nach Beispiel 2c dargestellten Aldehyds setzt man in Analogie zu Beispiel 1b unter Verwendung von Phenylmethoxyamin um und isoliert nach Aufarbeitung und Reinigung 79 mg (178 µmol, 75%) der Titelverbindung als farbloses Öl.
IR (Film): 3040, 2940, 2850, 1735, 1450, 1435, 1355, 1200, 1120, 1075, 1035, 1020, 970, 910, 865, 815 und 695 cm⁻¹.

### Beispiel 11:

### 5-{(E)-(1S,5S,6S,7R)-7-Hydroxy-6-[(E/Z)-1-naphtylmethoxyiminomethyl]-bicyclo[3.3.0]oct-3-yliden}-pentansäure:

80 mg (190 µmol) des nach Beispiel 11a dargestellten Esters verseift man in Analogie zu Beispiel 1 und isoliert nach Aufarbeitung und Reinigung 71 mg (174 µmol, 92%) der Titelverbindung als farbloses Öl.
IR (Film): 3600-2400, 3050, 2930, 1705, 1600, 1430, 1385, 1240, 1165, 1080, 1020, 915, 800, 790 und 775 cm⁻¹.

### Beispiel 11a:

### 5-{(E)-(1S,5S,6S,7R)-7-Hydroxy-6-[(E/Z)-1-naphtylmethoxyiminomethyl]-bicyclo[3.3.0]oct-3-yliden}-pentansäuremethylester:

109 mg (216 µmol) der nach Beispiel 11b dargestellten Verbindung setzt man in Analogie zu Beispiel 1a um und isoliert nach Aufarbeitung und Reinigung 80 mg (190 µmol, 88%) der Titelverbindung als farbloses Öl.
IR (Film): 3500-3200, 3050, 2930, 2860, 1735, 1600, 1425, 1385, 1240, 1165, 1080, 1020, 915, 805, 790 und 775 cm⁻¹.

### Beispiel 11b:

### 5-{(E)-(1S,5S,6S,7R)-7-(Tetrahydropryan-2-yloxy)-6-[(E/Z)-1-naphtylmethoxyiminomethyl]-bicyclo[3.3.0]oct-3-yliden}-pentansäuremethylester:

33 mg (236 µmol) des nach Beispiel 1c dargestellten Aldehyds setzt man in Analogie zu Beispiel 1b unter Verwendung von 1-Naphtylmethoxyamin um und isoliert nach Aufarbeitung und Reinigung 109 mg (216 µmol, 92%) der Titelverbindung als farbloses Öl.
IR (Film): 3040, 2940, 2850, 1735, 1595, 1450, 1435, 1350, 1200, 1160, 1120, 1075, 1030, 1020, 970, 910, 865, 820, 790 und 725 cm⁻¹.

### Beispiel 12:

### 5-{(Z)-(1S,5S,6S,7R)-7-Hydroxy-6-[(E/Z)-1-naphtylmethoxyiminomethyl]-bicyclo[3.3.0]oct-3-yliden}-pentansäure:

57 mg (135 µmol) des nach Beispiel 12a dargestellten Esters verseift man in Analogie zu Beispiel 1 und isoliert nach Aufarbeitung und Reinigung 54 mg (132 µmol, 98%) der Titelverbindung als farbloses Öl.
IR (Film): 3600-2500, 3050, 2940, 1705, 1600, 1240, 1080, 1000, 915, 800, 790 und 780 cm⁻¹.

### Beispiel 12a:

### 5-{(Z)-(1S,5S,6S,7R)-7-Hydroxy-6-[(E/Z)-1-naphtylmethoxyiminomethyl]-bicyclo[3.3.0]oct-3-yliden}-pentansäuremethylester:

97 mg (192 µmol) der nach Beispiel 12b dargestellten Verbindung setzt man in Analogie zu Beispiel 1a um und isoliert nach Aufarbeitung und Reinigung 57 mg (135 µmol, 70%) der Titelverbindung als farbloses Öl.
IR (Film): 3500-3200, 3040, 2950, 1735, 1595, 1240, 1080, 995, 915, 800, 790 und 780 cm⁻¹.

### Beispiel 12b:

### 5-{(Z)-(1S,5S,6S,7R)-7-(Tetrahydropyran-2-yloxy)-6-[(E/Z)-1-naphtylmethoxyiminomethyl]-bicyclo[3.3.0]oct-3-yliden}-pentansäuremethylester:

83 mg (236 µmol) des nach Beispiel 2d dargestellten Aldehyds setzt man in Analogie zu Beispiel 1b unter Verwendung von 1-Naphtylmethoxyamin um und isoliert nach Aufarbeitung und Reinigung 97 mg (192 µmol, 82%) der Titelverbindung als farbloses Öl.
IR (Film): 3040, 2960, 1735, 1600, 1235, 1080, 1000, 915, 865, 815, 800, 790 und 780 cm⁻¹.

### Beispiel 13:

### 5-{(E)-(1S,5S,6S,7R)-7-Hydroxy-6-[(E/Z)-diphenylmethoxyiminomethyl]-bicyclo[3.3.0]oct-3-yliden}-pentansäure:

69 mg (154 µmol) des nach Beispiel 13a dargestellten Esters verseift man in Analogie zu Beispiel 1 und isoliert nach Aufarbeitung und Reinigung 48 mg (110 µmol, 71%) der Titelverbindung als farbloses Öl.
IR (Film): 3600-2400, 3080, 3030, 2930, 2860, 1705, 1600, 1495, 1455, 1300, 1245, 1185, 1080, 1020, 935, 915, 745 und 700 cm⁻¹.

### Beispiel 13a:

### 5-{(E)-(1S,5S,6S,7R)-7-Hydroxy-6-[(E/Z)-diphenylmethoxyiminomethyl]-bicyclo[3.3.0]oct-3-yliden}-pentansäuremethylester:

100 mg (188 µmol) der nach Beispiel 13b dargestellten Verbindung setzt man in Analogie zu Beispiel 1a um und isoliert nach Aufarbeitung und Reinigung 69 mg (154 µmol, 82%) der Titelverbindung als farbloses Öl.
IR (Film): 3500-3200, 3080, 3030, 2940, 2870, 1730, 1600, 1495, 1455, 1300, 1245, 1185, 1075, 1020, 935, 915, 745 und 700 cm⁻¹.

### Beispiel 13b:

### 5-{(E)-(1S,5S,6S,7R)-7-(Tetrahydropyran-2-yloxy)-6-[(E/Z)-diphenylmethoxyiminomethyl]-bicyclo[3.3.0]oct-3-yliden}-pentansäuremethylester:

82 mg (234 µmol) des nach Beispiel 1d dargestellten Aldehyds setzt man in Analogie zu Beispiel 1b unter Verwendung von Diphenylmethoxyamin um und isoliert nach Aufarbeitung und Reinigung 109 mg (205 µmol, 88%) der Titelverbindung als farbloses Öl.
IR (Film): 3080, 3030, 2940, 2870, 1735, 1600, 1495, 1450, 1300, 1245, 1185, 1080, 1020, 935, 915, 865, 815, 745 und 700 cm⁻¹.

### Beispiel 14:

### 5-{(Z)-(1S,5S,6S,7R)-7-Hydroxy-6-[(E/Z)-diphenylmethoxyiminomethyl]-bicyclo[3.3.0]oct-3-yliden}-pentansäure:

147 mg (328 µmol) des nach Beispiel 14a dargestellten Esters verseift man in Analogie zu Beispiel 1 und isoliert nach Aufarbeitung und Reinigung 98 mg (226 µmol, 69%) der Titelverbindung als farbloses Öl.
IR (Film): 3600-2400, 3070, 3040, 2940, 1710, 1600, 1495, 1455, 1265, 1245, 1175, 1080, 1020, 935, 920, 745 und 700 cm⁻¹.

### Beispiel 14a:

### 5-{(Z)-(1S,5S,6S,7R)-7-Hydroxy-6-[(E/Z)-diphenylmethoxyiminomethyl]-bicyclo[3.3.0]oct-3-yliden}-pentansäuremethylester:

209 mg (393 µmol) der nach Beispiel 14b dargestellten Verbindung setzt man in Analogie zu Beispiel 1a um und isoliert nach Aufarbeitung und Reinigung 147 mg (328 µmol, 84%) der Titelverbindung als farbloses Öl.
IR (Film): 3500-3200, 3070, 3040, 2940, 1735, 1600, 1495, 1455, 1265, 1245, 1175, 1080, 1020, 935, 920, 745 und 700 cm⁻¹.

### Beispiel 14b:

### 5-{(Z)-(1S,5S,6S,7R)-7-(Tetrahydropyran-2-yloxy)-6-[(E/Z)-diphenylmethoxyiminomethyl]-bicyclo[3.3.0]oct-3-yliden}-pentansäuremethylester:

345 mg (984 µmol) des nach Beispiel 2d dargestellten Aldehyds setzt man in Analogie zu Beispiel 1b unter Verwendung von Diphenylmethoxyamin um und isoliert nach Aufarbeitung und Reinigung 426 mg (801 µmol, 81%) der Titelverbindung als farbloses Öl.
IR (Film): 3070, 3050, 2940, 2870, 1735, 1600, 1495, 1450, 1265, 1245, 1175, 1080, 1020, 935, 920, 865, 820, 745 und 695 cm⁻¹.

### Beispiel 15:

### 5-{((E)-1S,5S,6S,7R)-7-Hydroxy-6-[(E/Z)-3-(3-chlorphenyl)-ureidominomethyl]-bicyclo[3.3.0]oct-3-yliden}-pentansäure:

50 mg (115 µmol) der nach Beispiel 15a dargestellten Verbindung verseift man in Analogie zu Beispiel 1 und isoliert nach Aufarbeitung und Reinigung 25 mg (60 µmol, 52%) der Titelverbindung als kristallinen Feststoff.
¹H-NMR (CD₃OD): δ= 1,2-1,32(m,1H), 1,6-1,74 (m,2H), 2,0-2,6(m,13H), 3,9-4,04 (m,1H), 5,27-5,37 (m,1H), 7,02 (m,1H), 7,2 (d,1H), 7,25(d,1H), 7,38 (m,1H), 7,7(m,1H).

### Beispiel 15a:

### 5-{((E)-1S,5S,6S,7R)-7-Hydroxy-6-[(E/Z)-3-(3-chlorphenyl)-ureidoiminomethyl]-bicyclo[3.3.0]oct-3-yliden}-pentansäuremethylester:

81 mg (232 µmol) der nach Beispiel 1c dargestellten Verbindung setzt man in Analogie zu Beispiel 1a unter Verwendung von 4-(3-Chlorphenyl)-semicarbazid Hydrochlorid um und isoliert nach Aufarbeitung und Reinigung 50 mg (115 µmol, 50%) der Titelverbindung als farbloses Öl.
IR (Film): 3600-3000, 3360, 3110, 2940, 1735, 1690, 1590, 1530, 1480, 1425, 1305, 1225, 1095, 1015, 875, 775, 740 und 680 cm⁻¹.

### Beispiel 16:

### 5-{((Z)-1S,5S,6S,7R)-7-Hydroxy-6-[(E/Z)-3-(3-chlorphenyl)-ureidoiminomethyl]-bicyclo[3.3.0]oct-3-yliden}-pentansäure:

59 mg (136 µmol) der nach Beispiel 16a dargestellten Verbindung verseift man in Analogie zu Beispiel 1 und isoliert nach Aufarbeitung und Reinigung 53 mg (125 µmol, 92%) der Titelverbindung als farblosen Feststoff.
IR (KBr): 3600-2400, 3360, 3230, 2940, 1700, 1590, 1530, 1425, 1310, 1230, 1090, 1010, 870, 775 und 680 cm⁻¹.

### Beispiel 16a:

### 5-{((Z)-1S,5S,6S,7R)-7-Hydroxy-6-[(E/Z)-3-(3-chlorphenyl)-ureidoiminomethyl]-bicyclo[3.3.0]oct-3-yliden}-pentansäuremethylester:

100 mg (226 µmol) der nach Beispiel 2c dargestellten Verbindung setzt man in Analogie zu Beispiel 1b unter Verwendung von 4-(2-Chlorphenyl)-semicarbazid-Hydrochlorid um und isoliert nach Aufarbeitung und Reinigung 59 mg (136 µmol, 60%) der Titelverbindung als farbloses Öl.
IR (Film): 3600-3000, 3360, 3110, 2940, 1730, 1690, 1585, 1530, 1480, 1425, 1305, 1225, 1095, 1010, 875, 775, 740 und 680 cm⁻¹.

### Beispiel 17:

### 5-{((E)-1S,5S,6S,7R)-7-Hydroxy-6-[(E/Z)-3-(3,4-dichlorphenyl)-ureidoiminomethyl]-bicyclo[3.3.0]oct-3-yliden}-pentansäure:

26 mg (55 µmol) der nach Beispiel 17a dargestellten Verbindung verseift man in Analogie zu Beispiel 1 und isoliert nach Aufarbeitung und Reinigung 10 mg (22 µmol, 40%) der Titelverbindung als kristallinen Feststoff.
¹H-NMR (CD₃OD): δ= 1,2-1,34(m,1H), 1,6-1,74(m,2H), 1,96-2,6(m,13H), 3,9-4,04(m,1H), 5,3(m,1H), 7,2(d,1H), 7,4(m,2H), 7,87(m,1H).

### Beispiel 17a:

### 5-{((E)-1S,5S,6S,7R)-7-Hydroxy-6-[(E/Z)-3-(3,4-dichlorphenyl)-ureidoiminomethyl]-bicyclo[3.3.0]oct-3-yliden}-pentansäuremethylester:

81 mg (232 µmol) der nach Beispiel 1c dargestellten Verbindung setzt man in Analogie zu Beispiel 1b unter Verwendung von 4-(3,3-Dichlorphenyl)-semicarbazid Hydrochlorid um und isoliert nach Aufarbeitung und Reinigung 26 mg (55 µmol, 24%) der Titelverbindung als farbloses Öl.
IR (Film): 3600-3000, 3360, 3120, 2940, 1730, 1690, 1580, 1520, 1470, 1390, 1160, 1110, 1025, 865, 825, 770 und 740 cm⁻¹.

### Beispiel 18:

### 5-{((Z)-1S,5S,6S,7R)-7-Hydroxy-6-[(E/Z)-3-(3,4-dichlorphenyl)-ureidoiminomethyl]-bicyclo[3.3.0]oct-3-yliden}-pentansäure:

64 mg (137 µmol) der nach Beispiel 18a dargestellten Verbindung verseift man in Analogie zu Beispiel 1 und isoliert nach Aufarbeitung und Reinigung 48 mg (105 µmol, 77%) der Titelverbindung als farblosen Feststoff.
IR (KBr): 3600-2400, 3360, 3330, 2940, 1700, 1580, 1525, 1470, 1395, 1310, 1225, 1130, 1030, 870, 815 und 750 cm⁻¹.

### Beispiel 18a:

### 5-{((Z)-1S,5S,6S,7R)-7-Hydroxy-6-[(E/Z)-3-(3,4-dichlorphenyl)-ureidoiminomethyl]-bicyclo[3.3.0]oct-3-yliden}-pentansäuremethylester:

100 mg (226 µmol) der nach Beispiel 2c dargestellten Verbindung setzt man in Analogie zu Beispiel 1b unter Verwendung von 4-(3,4-Dichlorphenyl)-semicarbazid-Hydrochlorid um und isoliert nach Aufarbeitung und Reinigung mg ( µmol, %) der Titelverbindung als farbloses Öl.
IR (Film): 3600-3000, 3360, 3200, 3120, 2940, 1730, 1690, 1580, 1520, 1470, 1390, 1160, 1120, 1025, 865, 825, 770 und 740 cm⁻¹.

### Beispiel 19:

### 5-{((E)-1S,5S,6S,7R)-7-Hydroxy-6-[(E/Z)-3-(4-chlorphenyl)-ureidoiminomethyl]-bicyclo[3.3.0]oct-3-yliden}-pentansäure:

30 mg (63 µmol) der nach Beispiel 19a dargestellten Verbindung verseift man in Analogie zu Beispiel 1 und isoliert nach Aufarbeitung und Reinigung 12 mg (29 µmol, 45%) der Titelverbindung als kristallinen Feststoff.
¹H-NMR (CD₃OD): δ= 1,2-1,32(m,1H), 1,58-1,74(m,2H), 1,96-2,6(m,13H), 3,9-4,05 (m,1H), 5,32(m,1H), 7,17-7,3(m,3H), 7,5(d,2H).

### Beispiel 19a:

### 5-{((E)-1S,5S,6S,7R)-7-Hydroxy-6-[(E/Z)-3-(4-chlorphenyl)-ureidoiminomethyl]-bicyclo[3.3.0]oct-3-yliden}-pentansäuremethylester:

81 mg (232 µmol) der nach Beispiel 1c dargestellten Verbindung setzt man in Analogie zu Beispiel 1b unter Verwendung von 4-(4-Chlorphenyl)-semicarbazid Hydrochlorid um und isoliert nach Aufarbeitung und Reinigung 30 mg (63 µmol, 27%) der Titelverbindung als farbloses Öl.
IR (Film): IR (Film): 3600-3000, 3350, 3220, 2940, 1730, 1680, 1590, 1530, 1490, 1400, 1310, 1225, 1090, 825 und 735 cm⁻¹.

### Beispiel 20:

### 5-{((Z)-1S,5S,6S,7R)-7-Hydroxy-6-[(E/Z)-3-(4-chlorphenyl)-ureidoiminomethyl]-bicyclo[3.3.0]oct-3-yliden}-pentansäure:

61 mg (141 µmol) der nach Beispiel 20a dargestellten Verbindung verseift man in Analogie zu Beispiel 1 und isoliert nach Aufarbeitung und Reinigung 51 mg (122 µmol, 87%) der Titelverbindung als farblosen Feststoff.
IR (KBr): 3600-2400, 3360, 3240, 2940, 1700, 1590, 1530, 1490, 1405, 1310, 1230, 1090, 1010, 820 und 750 cm⁻¹.

### Beispiel 20a:

### 5-{((Z)-1S,5S,6S,7R)-7-Hydroxy-6-[(E/Z)-3-(4-chlorphenyl)-ureidoiminomethyl]-bicyclo[3.3.0]oct-3-yliden}-pentansäuremethylester:

100 mg (226 µmol) der nach Beispiel 2c dargestellten Verbindung setzt man in Analogie zu Beispiel 1b unter Verwendung von 4-(4-Chlorphenyl)-semicarbazid-Hydrochlorid um und isoliert nach Aufarbeitung und Reinigung 61 mg (141 µmol, 62%) der Titelverbindung als farbloses Öl.
IR (Film): 3600-3000, 3360, 3210, 2940, 1730, 1680, 1590, 1530, 1490, 1400, 1310, 1230, 1090, 825 und 735 cm⁻¹.

### Beispiel 21:

### 5-{((E)-1S,5S,6S,7R)-7-Hydroxy-6-[(E/Z)-3-(4-nitrophenyl)-ureidoiminomethyl]-bicyclo[3.3.0]oct-3-yliden}-pentansäure:

21 mg (47 µmol) der nach Beispiel 21a dargestellten Verbindung verseift man in Analogie zu Beispiel 1 und isoliert nach Aufarbeitung und Reinigung 6 mg (14 µmol, 30%) der Titelverbindung als kristallinen Feststoff.
¹H-NMR (CD₃OD): δ= 1,2-1,32(m,1H), 1,62-1,74(m,2H), 2-2,65(m,13H), 3,94-4,05(m, 1H), 5,32(m,1H), 7,22(d,1H), 7,79(d,2H), 8,18(d,1H).

### Beispiel 21a:

### 5-{((E)-1S,5S,6S,7R)-7-Hydroxy-6-[(E/Z)-3-(4-nitrophenyl)-ureidoiminomethyl]-bicyclo[3.3.0]oct-3-yliden}-pentansäuremethylester:

81 mg (232 µmol) der nach Beispiel 1c dargestellten Verbindung setzt man in Analogie zu Beispiel 1b unter Verwendung von 4-(4-Nitrophenyl)-semicarbazid Hydrochlorid um und isoliert nach Aufarbeitung und Reinigung 21 mg (47 µmol, 20%) der Titelverbindung als farbloses Öl.
IR (Film): 3600-3000, 3340, 3220, 3120, 2940, 1725, 1690, 1600, 1540, 1505, 1415, 1330, 1230, 1175, 1110, 995, 850 und 735 cm⁻¹.

### Beispiel 22:

### 5-{((Z)-1S,5S,6S,7R)-7-Hydroxy-6-[(E/Z)-3-(4-nitrophenyl)-ureidoiminomethyl]-bicyclo[3.3.0]oct-3-yliden}-pentansäure:

60 mg (135 µmol) der nach Beispiel 22a dargestellten Verbindung verseift man in Analogie zu Beispiel 1 und isoliert nach Aufarbeitung und Reinigung 37 mg (85 µmol, 63%) der Titelverbindung als blassgelbes Öl.
IR (Film): 3600-2400, 3350, 3210, 2940, 1700, 1600, 1540, 1505, 1410, 1330, 1240, 1180, 1110, 850 und 735 cm⁻¹.

### Beispiel 22a:

### 5-{((Z)-1S,5S,6S,7R)-7-Hydroxy-6-[(E/Z)-3-(4-nitrophenyl)-ureidoiminomethyl]-bicyclo[3.3.0]oct-3-yliden}-pentansäuremethylester:

100 mg (226 µmol) der nach Beispiel 2c dargestellten Verbindung setzt man in Analogie zu Beispiel 1b unter Verwendung von 4-(4-Nitrophenyl)-semicarbazid-hydrochlorid um und isoliert nach Aufarbeitung und Reinigung 60 mg (135 µmol, 60%) der Titelverbindung als blassgelbes Öl.
IR (Film): 3600-3000, 3340, 3210, 3120, 2940, 1720, 1690, 1600, 1540, 1505, 1415, 1330, 1235, 1175, 1110, 995, 850 und 735 cm⁻¹.

### Beispiel 23:

### 5-{((E)-1S,5S,6S,7R)-7-Hydroxy-6-[(E/Z)-3-phenyl-thioureido-iminomethyl]-bicyclo[3.3.0]oct-3-yliden}-pentansäure:

52 mg (125 µmol) der nach Beispiel 23a dargestellten Verbindung verseift man in Analogie zu Beispiel 1 und isoliert nach Aufarbeitung und Reinigung 16 mg (40 µmol, 32%) der Titelverbindung als farbloses Öl.
IR (CHCl₃): 3600-2400, 3410, 3330, 2950, 2860, 1710, 1595, 1530, 1495, 1430, 1315, 1250, 1180, 1125, 1075, 1030, 970 und 695 cm⁻¹.

### Beispiel 23a:

### 5-{((E)-1S,5S,6S,7R)-7-Hydroxy-6-[(E/Z)-3-phenyl-thioureido-iminomethyl]-bicyclo[3.3.0]oct-3-yliden}-pentansäuremethylester:

111 mg (222 µmol) der nach Beispiel 1c dargestellten Verbindung setzt man in Analogie zu Beispiel 1a um und isoliert nach Aufarbeitung und Reinigung 52 mg (125 µmol, 56%) der Titelverbindung als farbloses Öl.
IR (Film): 3600-3050, 3420, 3340, 3000, 2950, 2860, 1725, 1600, 1530, 1495, 1430, 1315, 1250, 1180, 1125, 1075, 1030, 970, und 695 cm⁻¹.

### Beispiel 23b:

### 5-{((E)-1S,5S,6S,7R)-7-(tetrahydropyran-2-yloxy)-6-[(E/Z)-3-phenylthioureidoiminomethyl]-bicyclo[3.3.0]oct-3-yliden}-pentansäuremethylester:

122 mg (350 µmol) der nach Beispiel 1c dargestellten Verbindung setzt man in Analogie zu Beispiel 1b unter Verwendung von 4-Phenyl-3-thiosemicarbazid um und isoliert nach Aufarbeitung und Reinigung 111 mg (222 µmol, 63%) der Titelverbindung als farbloses Öl.
IR (CHCl₃): 3420, 3340, 3000, 2950, 2860, 1725, 1595, 1530, 1495, 1430, 1315, 1250, 1180, 1125, 1075, 1030, 970, 870, 810 und 695 cm⁻¹.

### Beispiel 24:

### 5-{((Z)-1S,5S,6S,7R)-7-Hydroxy-6-[(E/Z)-3-phenyl-thioureidoiminomethyl]-bicyclo[3.3.0]oct-3-yliden}-pentansäure:

67 mg (161 µmol) der nach Beispiel 24a dargestellten Verbindung verseift man in Analogie zu Beispiel 1 und isoliert nach Aufarbeitung und Reinigung 31 mg (78 µmol, 48%) der Titelverbindung als farblosen Feststoff.
IR (CHCl₃): 3600-2600, 3330, 2940, 1705, 1595, 1535, 1445, 1320, 1260, 1070, 1040, 930 und 670 cm⁻¹.

### Beispiel 24a:

### 5-{((Z)-1S,5S,6S,7R)-7-Hydroxy-6-[(E/Z)-3-phenyl-thioureido-iminomethyl]-bicyclo[3.3.0]oct-3-yliden}-pentansäuremethylester:

100 mg (226 µmol) der nach Beispiel 2c dargestellten Verbindung setzt man in Analogie zu Beispiel 1b unter Verwendung von 4-Phenylthiosemicarbazid und Zugabe eines Äquivalentes p-Toluolsulfonsäure um und isoliert nach Aufarbeitung und Reinigung 67 mg (161 µmol, 71%) der Titelverbindung als blassgelbes Öl.
IR (Film): 3600-3000, 3310, 3040, 2940, 1725, 1595, 1530, 1495, 1430, 1310, 1255, 1195, 1080, 935, 750, 735 und 695 cm⁻¹.

### Beispiel 25:

### 5-{((E)-1S,5S,6S,7R)-7-Hydroxy-6-[(E/Z)-3-(2-chlorphenyl)-ureidoiminomethyl]-bicyclo[3.3.0]oct-3-yliden}-pentansäure:

69 mg (159 µmol) der nach Beispiel 25a dargestellten Verbindung verseift man in Analogie zu Beispiel 1 und isoliert nach Aufarbeitung und Reinigung 40 mg (95 µmol, 60%) der Titelverbindung als farbloses Öl.
¹H-NMR (CD₃OD): δ= 1,2-1,34(m,1H), 1,6-1,74(m,2H), 2-2,6(m,13H), 3,93-4,05(m,1H), 5,32(m,1H), 7,02(dt,1H), 7,22-7,34(m,2H), 7,41(dd,1H), 8,2(dd,1H).

### Beispiel 25a:

### 5-{((E)-1S,5S,6S,7R)-7-Hydroxy-6-[(E/Z)-3-(2-chlorphenyl)-ureidoiminomethyl]-bicyclo[3.3.0]oct-3-yliden}-pentansäuremethylester:

81 mg (232 µmol) der nach Beispiel 1c dargestellten Verbindung setzt man in Analogie zu Beispiel 1b unter Verwendung von 4-(2-Chlorphenyl)-semicarbazid Hydrochlorid um und isoliert nach Aufarbeitung und Reinigung 69 mg (159 µmol, 69%) der Titelverbindung als farbloses Öl.
IR (Film): 3600-3000, 3340, 3210, 3120, 2940, 1730, 1705, 1695, 1590, 1580, 1530, 1440, 1305, 1230, 1125, 1035, 935 und 750 cm⁻¹.

### Beispiel 26:

### 5-{((Z)-1S,5S,6S,7R)-7-Hydroxy-6-[(E/Z)-3-(2-chlorphenyl)-ureidoiminomethyl]-bicyclo[3.3.0]oct-3-yliden}-pentansäure:

114 mg (263 µmol) der nach Beispiel 26a dargestellten Verbindung verseift man in Analogie zu Beispiel 1 und isoliert nach Aufarbeitung und Reinigung 98 mg (232 µmol, 88%) der Titelverbindung als farblosen Feststoff.
IR (KBr): 3600-2400, 3340, 3210, 3120, 2940, 1700, 1580, 1530, 1440, 1300, 1225, 1125, 1035, 935 und 750 cm⁻¹.

### Beispiel 26a:

### 5-{((Z)-1S,5S,6S,7R)-7-Hydroxy-6-[(E/Z)-3-(2-chlorphenyl)-ureidoiminomethyl]-bicyclo[3.3.0]oct-3-yliden}-pentansäuremethylester:

125 mg (282 µmol) der nach Beispiel 2c dargestellten Verbindung setzt man in Analogie zu Beispiel 1b unter Verwendung von 4-(2-Chlorphenyl)-semicarbazid-Hydrochlorid um und isoliert nach Aufarbeitung und Reinigung 114 mg (263 µmol, 93%) der Titelverbindung als farbloses Öl.
IR (Film): 3600-3000, 3340, 3210, 3120, 2940, 1730, 1705, 1690, 1590, 1580, 1530, 1440, 1305, 1230, 1125, 1035, 935 und 750 cm⁻¹.

### Beispiel 27:

### 5-{((E)-1S,5S,6S,7R)-7-Hydroxy-6-[(E/Z)-3-(4-phenoxyphenyl)-thioureidoiminomethyl]-bicyclo[3.3.0]oct-3-yliden}-pentansäure:

71 mg (171 µmol) der nach Beispiel 27a dargestellten Verbindung verseift man in Analogie zu Beispiel 1 und isoliert nach Aufarbeitung und Reinigung 26 mg (53 µmol, 31%) der Titelverbindung als farbloses Öl.
IR (Film): 3600-2400, 3320, 3140, 3040, 2940, 2860, 1710, 1590, 1550-1450, 1435, 1400, 1280-1150, 1125, 1070, 1025, 970, 870, 840, 735 und 695 cm⁻¹.

### Beispiel 27a:

### 5-{((E)-1S,5S,6S,7R)-7-Hydroxy-6-[(E/Z)-3-(4-phenoxyphenyl)-thioureidoiminomethyl]-bicyclo[3.3.0]oct-3-yliden}-pentansäuremethylester:

132 mg (223 µmol) der nach Beispiel 27b dargestellten Verbindung setzt man in Analogie zu Beispiel 1b um und isoliert nach Aufarbeitung und Reinigung 71 mg (171 µmol, 77%) der Titelverbindung als farbloses Öl.
IR (Film): 3600-3000, 3330, 3140, 3040, 2940, 2860, 1730, 1590, 1550-1470, 1290-1150, 1125, 1070, 1025, 970, 840, 735 und 695 cm⁻¹.

### Beispiel 27b:

### 5-{((E)-1S,5S,6S,7R)-7-(tetrahydropyran-2-yloxy)-6-[(E/Z)-3-(4-phenoxyphenyl)-thioureidoiminomethyl]-bicyclo[3.3.0]oct-3-yliden}-pentansäuremethylester:

122 mg (350 µmol) der nach Beispiel 1c dargestellten Verbindung setzt man in Analogie zu Beispiel 1b unter Verwendung von 4-(4-Phenoxyphenyl)-semicarbazid um und isoliert nach Aufarbeitung und Reinigung 132 mg (223 µmol, 64%) der Titelverbindung als farbloses Öl.
IR (Film): 3320, 3130, 3040, 2940, 2850, 1730, 1590, 1550-1470, 1270-1150, 1125, 1070, 1025, 970, 870, 840, 810, 735 und 690 cm⁻¹.

### Beispiel 28:

### 5-{((Z)-1S,5S,6S,7R)-7-Hydroxy-6-[(E/Z)-3-(4-phenoxyhenyl)-thioureidoiminomethyl]-bicyclo[3.3.0]oct-3-yliden}-pentansäure:

60 mg (118 µmol) der nach Beispiel 28a dargestellten Verbindung verseift man in Analogie zu Beispiel 1 und isoliert nach Aufarbeitung und Reinigung 17 mg (35 µmol, 30%) der Titelverbindung als farbloses Öl.
IR (Film): 3600-2400, 3320, 3050, 2940, 1700, 1590, 1540, 1500, 1480, 1400, 1310, 1225, 1165, 1070, 870, 845, 740 und 690 cm⁻¹.

### Beispiel 28a:

### 5-{((Z)-1S,5S,6S,7R)-7-Hydroxy-6-[(E/Z)-3-(4-phenoxyphenyl)-thioureidoiminomethyl]-bicyclo[3.3.0]oct-3-yliden}-pentansäuremethylester:

125 mg (282 µmol) der nach Beispiel 2c dargestellten Verbindung setzt man in Analogie zu Beispiel 1b unter Verwendung von 4-(4-Phenoxyphenyl)-semicarbazid-Hydrochlorid um und isoliert nach Aufarbeitung und Reinigung 60 mg (118 µmol, 42%) der Titelverbindung als blassgelbes Öl.
IR (Film): 3600-3100, 3320, 3050, 2940, 1725, 1590, 1540, 1500, 1485, 1400, 1310, 1230, 1165, 1070, 870, 845, 740 und 690 cm⁻¹.

### Beispiel 29:

### 5-{((Z)-1S,5S,6S)-6-[(E/Z)-3-Phenylureidoiminomethyl]-bicyclo[3.3.0]oct-3-yliden}-pentansäure:

85 mg (227 µmol) der nach Beispiel 29a dargestellten Verbindung verseift man in Analogie zu Beispiel 1 und isoliert nach Aufarbeitung und Reinigung 65 mg (176 µmol, 77%) der Titelverbindung als kristallinen Feststoff.
¹H-NMR (CD₃OD): δ= 1,25-1,48 (m,1H), 1,5-1,74 (m,3H), 1,85-2,6(m,13H), 5,2-5,3 (m,1H), 7,05 (t,1H), 7,2 (d,1H), 7,28(t,1H), 7,48 (d,1H).

### Beispiel 29a:

### 5-{((Z)-1S,5S,6S)-6-[(E/Z)-3-Phenylureidoiminomethyl]-bicyclo[3.3.0]oct-3-yliden}-pentansäuremethylester:

67 mg (267 µmol) der nach Beispiel 29b dargestellten Verbindung setzt man in Analogie zu Beispiel 1b unter Verwendung von 4-Phenylsemicarbazid um und isoliert nach Aufarbeitung und Reinigung 87 mg (227 µmol, 85%) der Titelverbindung als farbloses Öl.
IR (Film): 3370, 3200, 3100, 2940, 2860, 1735, 1685, 1590, 1530, 1445, 1310, 1230, 1195, 1170, 1130, 755 und 690 cm⁻¹.

### Beispiel 29b:

### 5-{((Z)-1S,5S,6S)-6-Formyl-bicyclo[3.3.0]oct-3-yliden}-pentansäuremethylester:

273 mg (1,08 mmol) der nach Beispiel 29c dargestellten Verbindung oxidiert man in Analogie zu Beispiel 1c und isoliert nach Aufarbeitung 275 mg der Titelverbindung als farbloses Öl, das ohne Reinigung weiter umgesetzt wird.

### Beispiel 29c:

### 5-{((Z)-1S,5S,6S)-6-Hydroxymethyl-bicyclo[3.3.0]oct-3-yliden}-pentansäuremethylester:

531 mg (1,08 mmol) der nach Beispiel 29d dargestellten Verbindung setzt man in Analogie zu Beispiel 1d um und isoliert nach Aufarbeitung und Reinigung 273 mg (1,08 mmol, 100%) der Titelverbindung als farbloses Öl.
IR (Film): 3600-3200, 2940, 2860, 1735, 1435, 1245, 1225, 1170 und 1030 cm⁻¹.

### Beispiel 29d:

### 5-{((Z)-1S,5S,6S)-6-tert.-Butyl-diphenylsilyloxymethyl-bicyclo[3.3.0]oct-3-yliden}-pentansäuremethylester:

755 mg (1,14 mmol) des nach Beispiel 29e dargestellten Tosylates löst man in 10 ml Dimethoxyethan, versetzt mit 760 mg Zinkstaub, 880 mg Natriumiodid, 670 µl Wasser und erhitzt 9 Stunden unter Rückfluß. Nach dem Erkalten filtriert man, wäscht mit Diethylether und die vereinigten organischen Phasen mit 10%iger Natriumthiosulfatlösung, Wasser und gesättigter Natriumchloridlösung und trocknet über Magnesiumsulfat. Die nach Filtration und Lösungsmittelabzug erhaltene Titelverbindung (531 mg, 1,08 mmol, 95%) setzt man ohne Reinigung weiter um.
IR (Film): 3070, 3040, 2940, 2850, 1735, 1590, 1425, 1245, 1165, 1110, 820, 740 und 700 cm⁻¹.

### Beispiel 29e:

### 5-{((Z)-1S,5S,6S,7R)-7-(Toluolsulfonyloxy)-6-tert.-butyl-diphenylsilyloxymethyl-bicyclo[3.3.0]oct-3-yliden}-pentansäuremethylester:

600 mg (1,18 mmol) des nach Beispiel 29f dargestellten Alkohols löst man in 2 ml wasserfreiem Pyridin, versetzt mit der Lösung von 936 mg p-Toluolsulfonsäurechlorid in 2 ml wasserfreiem Pyridin und erhitzt 2 Stunden unter einer Atmosphäre aus trockenem Argon auf 55°C. Nach dem Erkalten gießt man auf eine 2n Salzsäure, extrahiert mit Diethylether, wäscht die vereinigten organischen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an ca. 100 ml feinem Kieselgel unter Verwendung eines Gradientensystems aus n-Hexan und Ethylacetat. Isoliert werden 755 mg (1,14 mmol, 97%) der Titelverbindung als farbloses Öl.
IR (Film): 3070, 3040, 2940, 2850, 1735, 1600, 1425, 1360, 1190, 1175, 1110, 960, 935, 860, 820, 740, 705 und 665 cm⁻¹.

### Beispiel 29f:

### 5-{((Z)-1S,5S,6S,7R)-7-Hydroxy-6-tert.-butyl-diphenylsilyloxymethyl-bicyclo[3.3.0]oct-3-yliden}-pentansäuremethylester:

997 mg (1,69 mmol) der nach Beispiel 2e dargestellten Verbindung setzt man in Analogie zu Beispiel 1a um und isoliert nach Aufarbeitung und Reinigung 863 mg (1,69 mmol, 100%) der Titelverbindung als farbloses Öl.
IR (Film): 3600-3200, 3070, 3050, 2940, 2850, 2735, 1590, 1425, 1240, 1110, 820, 740 und 705 cm⁻¹.

### Beispiel 30:

### 5-{((Z)-1S,5S,6S)-6-[(E/Z)-3-(4-Chlorphenyl)-ureidoiminomethyl]-bicyclo[3.3.0]oct-3-yliden}-pentansäure:

62 mg (148 µmol) der nach Beispiel 30a dargestellten Verbindung verseift man in Analogie zu Beispiel 1 und isoliert nach Aufarbeitung und Reinigung 45 mg (112 µmol, 72%) der Titelverbindung als kristallinen Feststoff.
¹H-NMR (CD₃OD): δ= 1,24-1,38(m,1H), 1,5-1,74 (m,3H), 1,85-2,6(m,13H), 5,2-5,3 (m,1H), 7,19 (d,1H), 7,27(d,2H), 7,51 (d,2H).

### Beispiel 30a:

### 5-{((Z)-1S,5S,6S)-6-[(E/Z)-3-(4-Chlorphenyl)-ureidoiminomethyl]-bicyclo[3.3.0]oct-3-yliden}-pentansäuremethylester:

67 mg (267 µmol) der nach Beispiel 29b dargestellten Verbindung setzt man in Analogie zu Beispiel 1b unter Verwendung von 4-(4-Chlorphenyl)-semicarbazid Hydrochlorid um und isoliert nach Aufarbeitung und Reinigung 65 mg (156 µmol, 58%) der Titelverbindung als farbloses Öl.
IR (Film): 3360, 3200, 3200, 2940, 2860, 1730, 1685, 1590, 1525, 1490, 1435, 1400, 1310, 1280, 1230, 1170, 1130, 1090, 1010, 870, 825 und 745 cm⁻¹.

### Beispiel 31:

### 5-{((Z)-1S,5S,6S)-6-[(E/Z)-3-(3,4-Dichlorphenyl)-ureidoiminomethyl]-bicyclo[3.3.0]oct-3-yliden}-pentansäure:

87 mg (192 µmol) der nach Beispiel 31a dargestellten Verbindung verseift man in Analogie zu Beispiel 1 und isoliert nach Aufarbeitung und Reinigung 71 mg (163 µmol, 85%) der Titelverbindung als kristallinen Feststoff.
¹H-NMR (CD₃OD): δ= 1,25-1,38(m,1H), 1,5-1,74 (m,3H), 1,85-2,6(m,13H), 5,2-5,3 (m,1H), 7,19 (d,1H), 7,41(m,2H), 7,9 (m,1H).

### Beispiel 31a:

### 5-{((Z)-1S,5S,6S)-6-[(E/Z)-3-(3,4-Dichlorphenyl)-ureidoiminomethyl]-bicyclo[3.3.0]oct-3-yliden}-pentansäuremethylester:

67 mg (267 µmol) der nach Beispiel 29b dargestellten Verbindung setzt man in Analogie zu Beispiel 1b unter Verwendung von 4-(3,4-Dichlorphenyl)-semicarbazid Hydrochlorid um und isoliert nach Aufarbeitung und Reinigung 87 mg (192 µmol, 72%) der Titelverbindung als farbloses Öl.
IR (Film): 3360, 3200, 3110, 2940, 2860, 1730, 1690, 1575, 1520, 1475, 1390, 1295, 1225, 1195, 1170, 1130, 1025, 875, 815, 745 und 690 cm⁻¹.

### Beispiel 32:

### 5-{((Z)-1S,5S,6S)-6-[(E/Z)-3-(4-Nitrophenyl)-ureidoiminomethyl]-bicyclo[3.3.0]oct-3-yliden}-pentansäure:

98 mg (229 µmol) der nach Beispiel 32a dargestellten Verbindung verseift man in Analogie zu Beispiel 1 und isoliert nach Aufarbeitung und Reinigung 84 mg (203 µmol, 87%) der Titelverbindung als kristallinen Feststoff.
¹H-NMR (CD₃OD): δ= 1,25-1,4(m,1H), 1,5-1,74 (m,3H), 1,87-2,6(m,13H), 5,2-5,3 (m,1H), 7,22 (d,1H), 7,8(d,2H), 8,19 (d,2H).

### Beispiel 32a:

### 5-{((Z)-1S,5S,6S)-6-[(E/Z)-3-(4-Nitrophenyl)-ureidoiminomethyl]-bicyclo[3.3.0]oct-3-yliden}-pentansäuremethylester:

67 mg (267 µmol) der nach Beispiel 29b dargestellten Verbindung setzt man in Analogie zu Beispiel 1b unter Verwendung von 4-(4-Nitrophenyl)-semicarbazid Hydrochlorid um und isoliert nach Aufarbeitung und Reinigung 100 mg (233 µmol, 87%) der Titelverbindung als farbloses Öl.
IR (Film): 3340, 3200, 3110, 2940, 2860, 1725, 1690, 1600, 1535, 1505, 1410, 1330, 1235, 1175, 1100, 995, 850, 750 und 690 cm⁻¹.

### Beispiel 33:

### 5-{((E)-1S,5S,6S)-6-[(E/Z)-3-Phenylureidoiminomethyl]-bicyclo[3.3.0]oct-3-yliden}-pentansäure:

52 mg (136 µmol) der nach Beispiel 33a dargestellten Verbindung verseift man in Analogie zu Beispiel 1 und isoliert nach Aufarbeitung und Reinigung 29 mg (78 µmol, 58%) der Titelverbindung als farbloses Öl.
¹H-NMR (CD₃OD): δ= 1,25-1,4(m,2H), 1,5-1,72 (m,3H), 1,85-2,7(m,12H), 5,2-5,3 (m,1H), 7,04 (t,1H), 7,18 (d,1H), 7,29 (t,2H), 7,47 (d,2H).

### Beispiel 33a:

### 5-{((E)-1S,5S,6S)-6-[(E/Z)-3-Phenylureidoiminomethyl]-bicyclo[3.3.0]oct-3-yliden}-pentansäuremethylester:

50 mg (200 µmol) der nach Beispiel 33b dargestellten Verbindung setzt man in Analogie zu Beispiel 1b um und isoliert nach Aufarbeitung und Reinigung 52 mg (136 µmol, 68%) der Titelverbindung als farbloses Öl.
IR (Film): 3370, 3200, 3100, 2940, 2860, 1730, 1685, 1595, 1530, 1445, 1315, 1230, 1170, 1130, 755, 735 und 695 cm⁻¹.

### Beispiel 33b:

### 5-{((E)-1S,5S,6S)-6-Formyl-bicyclo[3.3.0]oct-3-yliden}-pentans äuremethylester:

456 mg (1,81 mmol) der nach Beispiel 33c dargestellten Verbindung setzt man in Analogie zu Beispiel 1c um und isoliert nach Aufarbeitung und Reinigung 458 mg der Titelverbindung, die man ohne Reinigung weiter umsetzt, als farbloses Öl.

### Beispiel 33c:

### 5-{((E)-1S,5S,6S)-6-Hydroxymethyl-bicyclo[3.3.0]oct-3-yliden}-pentansäuremethylester:

895 mg (1,82 mmol) der nach Beispiel 33d dargestellten Verbindung setzt man in Analogie zu Beispiel 1d um und isoliert nach Aufarbeitung und Reinigung 456 mg (1,81 mmol, 99%) der Titelverbindung als farbloses Öl.
IR (Film): 3600-3100, 2940, 2850, 1735, 1435, 1245, 1225, 1170 und 1030 cm⁻¹.

### Beispiel 33d:

### 5-{((E)-1S,5S,6S)-6-tert.-Butyl-diphenylsilyloxymethyl-bicyclo[3.3.0]oct-3-yliden}-pentansäuremethylester:

1,28 g (1,94 mmol) der nach Beispiel 33e dargestellten Verbindung setzt man in Analogie zu Beispiel 29d um und isoliert nach Aufarbeitung und Reinigung 895 mg (1,82 mmol, 94%) der Titelverbindung als farbloses Öl.
IR (Film): 3070, 3050, 2940, 2950, 1740, 1590, 1425, 1245, 1165, 1110, 820, 740 und 700 cm⁻¹.

### Beispiel 33e:

### 5-{((E)-1S,5S,6S,7R)-7-(Toluolsulfonyloxy)-6-tert.-butyl-diphenylsilyloxymethyl-bicyclo[3.3.0]oct-3-yliden}-pentansäuremethylester:

1,05 g (2,06 mmol) der nach Beispiel 33f dargestellten Verbindung setzt man in Analogie zu Beispiel 29e um und isoliert nach Aufarbeitung und Reinigung 1,28 g (1,94 mmol, 94%) der Titelverbindung als farbloses Öl.
IR (Film): 3070, 3050, 2940, 2850, 1735, 1595, 1425, 1360, 1185, 1175, 1110, 960, 940, 840, 815, 740, 700 und 665 cm⁻¹.

### Beispiel 33f:

### 5-{((E)-1S,5S,6S,7R)-7-Hydroxy-6-tert.-butyl-diphenylsilyloxymethyl-bicyclo[3.3.0]oct-3-yliden}-pentansäuremethylester:

3,84 g (6,37 mmol) der nach Beispiel 1e dargestellten Verbindung setzt man in Analogie zu Beispiel 1a um und isoliert nach Aufarbeitung und Reinigung 2,98 g (5,88 mmol, 92%) der Titelverbindung als farbloses Öl.
IR (Film): 3600-3100, 3070, 3050, 2940, 2850, 1735, 1590, 1425, 1245, 1170, 1110, 820, 740 und 705 cm⁻¹.

### Beispiel 34:

### 5-{((E)-1S,5S,6S)-6-[(E/Z)-3-(4-Chlorphenyl)-ureidoiminomethyl]-bicyclo[3.3.0]oct-3-yliden}-pentansäure:

35 mg (123 µmol) der nach Beispiel 34a dargestellten Verbindung verseift man in Analogie zu Beispiel 1 und isoliert nach Aufarbeitung und Reinigung 17 mg (42 µmol, 34%) der Titelverbindung als farbloses Öl.
¹H-NMR (CDCl₃): δ= 1,4-2,88(m,17H), 5,25-5,35(m,1H), 6,43(d,0,7H), 7,08(d,0,3H), 7,26 (d,2H), 7,44(d,2H), 8,02(s,NH), 8,24(s,NH), 9,08(s,NH), 10,4(s,NH).

### Beispiel 34a:

### 5-{((E)-1S,5S,6S)-6-[(E/Z)-3-(4-Chlorphenyl)-ureidoiminomethyl]-bicyclo[3.3.0]oct-3-yliden}-pentansäuremethylester:

50 mg (200 µmol) der nach Beispiel 33b dargestellten Verbindung setzt man in Analogie zu Beispiel 1b unter Verwendung von 4-(4-Chlorphenyl)-semicarbazidhydrochlorid um und isoliert nach Aufarbeitung und Reinigung 35 mg (123 µmol, 62%) der Titelverbindung als farbloses Öl.
IR (Film): 3370, 3200, 3100, 2940, 2860, 1725, 1685, 1590, 1525, 1490, 1405, 1310, 1230, 1175, 1125, 1090, 825 und 735 cm⁻¹.

### Beispiel 35:

### 5-{((E)-1S,5S,6S)-6-[(E/Z)-3-(3,4-Dichlorphenyl)-ureidoiminomethyl]-bicyclo[3.3.0]oct-3-yliden}-pentansäure:

44 mg (138 µmol) der nach Beispiel 35a dargestellten Verbindung verseift man in Analogie zu Beispiel 1 und isoliert nach Aufarbeitung und Reinigung 22 mg (50 µmol, 36%) der Titelverbindung als farbloses Öl.
¹H-NMR (CDCl₃): δ= 1,4-2,86(m,17H), 5,25-5,35(m,1H), 6,45(d,0,7H), 7,08(d,0,3H), 7,34 (m,2H), 7,72(m,1H), 8,05s,(NH), 8,28(s,NH), 9,13(s,NH), 10,43(s,NH).

### Beispiel 35a:

### 5-{((E)-1S,5S,6S)-6-[(E/Z)-3-(3,4-Dichlorphenyl)-ureidoiminomethyl]-bicyclo[3.3.0]oct-3-yliden}-pentansäuremethylester:

50 mg (200 µmol) der nach Beispiel 33b dargestellten Verbindung setzt man in Analogie zu Beispiel 1b unter Verwendung von 4-(3,4-Dichlorphenyl)-semicarbazidhydrochlorid um und isoliert nach Aufarbeitung und Reinigung 44 mg (138 µmol, 69%) der Titelverbindung als farbloses Öl.
IR (Film): 3370, 3200, 3110, 2940, 2860, 1725, 1690, 1580, 1520, 1475, 1390, 1295, 1260, 1130, 1025, 875, 810, 735 und 705 cm⁻¹.

### Beispiel 36:

### 5-{((E)-1S,5S,6S)-6-[(E/Z)-3-(4-Nitrophenyl)-ureidoiminomethyl]-bicyclo[3.3.0]oct-3-yliden}-pentansäure:

26 mg (61 µmol) der nach Beispiel 36a dargestellten Verbindung verseift man in Analogie zu Beispiel 1 und isoliert nach Aufarbeitung und Reinigung 12 mg (29 µmol, 47%) der Titelverbindung als farbloses Öl.
¹H-NMR (CDCl₃): δ= 1,45-2,88(m,17H), 5,25-5,35 (m,1H), 6,5(d,0,65H), 7,12(d,0,35H), 7,68(d,2H), 8,2(m,2H), 8,41(s,NH), 8,63(s,NH), 9,23(s,NH), 10,54(s,NH).

### Beispiel 36a:

### 5-{((E)-1S,5S,6S)-6-[(E/Z)-3-(4-Nitrophenyl)-ureidoiminomethyl]-bicyclo[3.3.0]oct-3-yliden}-pentansäuremethylester:

50 mg (200 µmol) der nach Beispiel 33b dargestellten Verbindung setzt man in Analogie zu Beispiel 1b unter Verwendung von 4-(4-Nitrophenyl)-semicarbazidhydrochlorid um und isoliert nach Aufarbeitung und Reinigung 26 mg (61 µmol, 31%) der Titelverbindung als farbloses Öl.
IR (Film): 3350, 3200, 3110, 2940, 2860, 1725, 1695, 1600, 1535, 1505, 1410, 1330, 1235, 1175, 1110, 855 und 750 cm⁻¹.

### Beispiel 37:

### 5-{((E)-1S,5S,6S)-6-[(E/Z)-3-(3-Chlorphenyl)-ureidoiminomethyl]-bicyclo[3.3.0]oct-3-yliden}-pentansäure:

34 mg (119 µmol) der nach Beispiel 37a dargestellten Verbindung verseift man in Analogie zu Beispiel 1 und isoliert nach Aufarbeitung und Reinigung 18 mg (45 µmol, 37%) der Titelverbindung als farbloses Öl.
¹H-NMR (CDCl₃): δ= 1,4-2,86(m,17H), 5,25-5,35(m,1H), 6,45(d,0,7H), 7,0-7,08(m,1H), 7,09(d,0,3H), 7,19-7,27(m,1H), 7,3-7,37(m,1H), 7,62(m,1H), 8,03(s,NH), 8,26(s,NH), 9,15(s,NH), 10,4(s,NH).

### Beispiel 37a:

### 5-{((E)-1S,5S,6S)-6-[(E/Z)-3-(3-Chlorphenyl)-ureidoiminomethyl]-bicyclo[3.3.0]oct-3-yliden}-pentansäuremethylester:

50 mg (200 µmol) der nach Beispiel 33b dargestellten Verbindung setzt man in Analogie zu Beispiel 1b unter Verwendung von 4-(3-Chlorphenyl)-semicarbazidhydrochlorid um und isoliert nach Aufarbeitung und Reinigung 34 mg (119 µmol, 60%) der Titelverbindung als farbloses Öl.
IR (Film): 3360, 3200, 3100, 2940, 2860, 1725, 1685, 1585, 1525, 1480, 1425, 1300, 1225, 1170, 1125, 1010, 875, 775, 745 und 680 cm⁻¹.

### Beispiel 38:

### 4-{((E)-1S,5S,6S,7R)-7-Hydroxy-6-[(E/Z)-3-phenylureidoiminomethyl]-bicyclo[3.3.0]oct-3-yliden}-butansäure:

35 mg (91 µmol) der nach Beispiel 38a dargestellten Verbindung verseift man in Analogie zu Beispiel 1 und isoliert nach Aufarbeitung und Reinigung 21 mg (55 µmol, 61%) der Titelverbindung als farbloses Öl.
¹H-NMR (CDCl₃): δ= 1,2-1,45(m,1H) 1,95-2,77(m,13H), 3,9-4,03(m,1H), 5,2-5,35(m,1H), 5,4-6,6(1H,COOH), 6,48(d,0,35H), 7,02(t,1H), 7,09(d,0,65H), 7,2-7,55(m,4H), 8,16(s,NH), 8,22(s,NH), 9,8(s,NH), 9,95(s,NH).

### Beispiel 38a:

### 4-{((E)-1S,5S,6S,7R)-7-Hydroxy-6-[(E/Z)-3-phenylureidoiminomethyl]-bicyclo[3.3.0]oct-3-yliden}-butansäuremethylester:

Die Lösung von 80 mg (238 µmol) des nach Beispiel 38b dargestellten Aldehyds in 2 ml wasserfreiem Ethanol versetzt man mit 48 mg 4-Phenylsemicarbazid, 18 mg Pyridinium-p-toluolsulfonat und erwärmt 6 Stunden unter einer Atmosphäre aus trockenem Argon auf 55°C. Die Aufarbeitung erfolgt wie in Beispiel 1a beschrieben. Nach Reinigung isoliert man 56 mg (145 µmol, 61%) der Titelverbindung als farbloses Öl. IR (Film): 3600-3100, 3360, 3100, 2940, 2850, 1730, 1680, 1590, 1530, 1445, 1325, 1230, 1175, 1130, 1030, 940, 755 und 690 cm⁻¹.

### Beispiel 38b:

### 4-{((E)-1S,5S,6R,7R)-7-(Tetrahydropyran-2-yloxy)-6-formyl-bicyclo[3.3.0]oct-3-yliden}-butansäuremethylester:

600 mg (1,77 mmol) der nach Beispiel 38c dargestellten Verbindung setzt man in Analogie zu Beispiel 1c um und isoliert nach Aufarbeitung 607mg der Titelverbindung als farbloses Öl, das man ohne Reinigung weiter umsetzt.

### Beispiel 38c:

### 4-{((E)-1S,5S,6S,7R)-7-(Tetrahydropyran-2-yloxy)-6-hydroxymethyl-bicyclo[3.3.0]oct-3-yliden}-butansäuremethylester:

4,11 g (7,3 mmol) der nach Beispiel 38d dargestellten Verbindung setzt man in Analogie zu Beispiel 1d um und isoliert nach Aufarbeitung und Reinigung 2,36 g (6,97 mmol, 96%) der Titelverbindung als farbloses Öl.
IR (Film): 3600-3200, 2940, 2860, 1735, 1435, 1350, 1255, 1200, 1170, 1135, 1075, 1020, 975, 865 und 810 cm⁻¹.

### Beispiel 38d:

### 4-{((E)-1S,5S,6S,7R)-7-(Tetrahydropyran-2-yloxy)-6-tert.-butyl-diphenylsilyloxymethyl-bicyclo[3.3.0]oct-3-yliden}-butansäuremethylester:

9,54 g (17 mmol) der nach Beispiel 38e dargestellten Verbindung A setzt man in Analogie zu Beispiel 1e um und isoliert nach Aufarbeitung und Reinigung 8,39 g (14,5 mmol, 86%) der Titelverbindung als farbloses Öl.
IR (Film): 3070, 3050, 2940, 2850, 1740, 1425, 1350, 1255, 1130, 1110, 1075, 1035, 1020, 975, 820, 740 und 705 cm⁻¹.

### Beispiel 38e:

### 4-{((E)-1S,5S,6S,7R)-7-(Tetrahydropyran-2-yloxy)-6-tert.-butyl-diphenylsilyloxymethyl-bicyclo[3.3.0]oct-3-yliden}-butansäure (A) und 4-[((Z)-1S,5S,6S,7R)-7-(Tetrahydropyran-2-yloxy)-6-tert.-butyl-diphenylsilyloxymethyl-bicyclo[3.3.0]oct-3-yliden}-butansäure (B):

19,1 g (38,8 mmol) der nach Beispiel 1g dargestellten Verbindung setzt man in Analogie zu Beispiel 1f um und isoliert nach Aufarbeitung und Reinigung 9,54 g (17 mmol, 44%) der Titelverbindung A, 5,57g (9,9 mmol, 26%) der Titelverbindung B sowie 3,78g (6,72 mmol, 17%) einer Mischfraktion aus A und B, jeweils als farbloses Öl.
IR (Film) von A und B: 3600-2400, 3070, 3050, 3010, 2940, 2850, 1705, 1425, 1355, 1200, 1125, 1110, 1075, 1020, 865, 815, 740 und 695 cm⁻¹.

### Beispiel 39:

### 4-{((Z)-1S,5S,6S,7R)-7-Hydroxy-6-[(E/Z)-3-phenylureidoiminomethyl]-bicyclo[3.3.0]oct-3-yliden}-butansäure:

54 mg (140 µmol) der nach Beispiel 39a dargestellten Verbindung verseift man in Analogie zu Beispiel 1 und isoliert nach Aufarbeitung und Reinigung 25 mg (67 µmol, 48%) der Titelverbindung als farblosen Feststoff.
¹H-NMR (CD₃OD): δ= 1,2-1,32(m,1H), 1,98-2,57(m,13H), 3,98(m,1H), 5,28-5,36(m,1H), 7,03(t,1H), 7,22(d,1H), 7,28(t,2H), 7,49(d,2H).

### Beispiel 39a:

### 4-{((Z)-1S,5S,6S,7R)-7-Hydroxy-6-[(E/Z)-3-phenylureidoiminomethyl]-bicyclo[3.3.0]oct-3-yliden}-butansäuremethylester:

120 mg (358 µmol) der nach Beispiel 39b dargestellten Verbindung setzt man in Analogie zu Beispiel 38a um und isoliert nach Aufarbeitung und Reinigung 54 mg (140 µmol, 39%) der Titelverbindung als farbloses Öl.
IR (Film): 3600-3000, 3360, 3210, 3100, 2940, 2840, 1725, 1680, 1590, 1530, 1445, 1320, 1230, 1170, 1130, 755, 735 und 690 cm⁻¹.

### Beispiel 39b:

### 4-{((Z)-1S,5S,6R,7R)-7-(Tetrahydropyran-2-yloxy)-6-formyl-bicyclo[3.3.0]oct-3-yliden}-butansäuremethylester:

600 mg (1,77 mmol) der nach Beispiel 39c dargestellten Verbindung setzt man in Analogie zu Beispiel 1c um und isoliert nach Aufarbeitung 602 mg der Titelverbindung als farbloses Öl, das man ohne Reinigung weiter umsetzt.

### Beispiel 39c:

### 4-{((Z)-1S,5S,6S,7R)-7-(Tetrahydropyran-2-yloxy)-6-hydroxymethyl-bicyclo[3.3.0]oct-3-yliden}-butansäuremethylester:

2,63 g (4,67 mmol) der nach Beispiel 39d dargestellten Verbindung setzt man in Analogie zu Beispiel 1d um und isoliert nach Aufarbeitung und Reinigung 1,58 g (4,67 mmol, 100%) der Titelverbindung als farbloses Öl.
IR (Film): 3600-3100, 2940, 2860, 1735, 1435, 1350, 1255, 1200, 1165, 1130, 1075, 1020 und 975 cm⁻¹.

### Beispiel 39d:

### 4-{((Z)-1S,5S,6S,7R)-7-(Tetrahydropyran-2-yloxy)-6-tert.-butyl-diphenylsilyloxymethyl-bicyclo[3.3.0]oct-3-yliden}-butansäuremethylester:

5,57 g (9,9 mmol) der nach Beispiel 38e dargestellten Verbindung B setzt man in Analogie zu Beispiel 1e um und isoliert nach Aufarbeitung und Reinigung 5,1 g (8,84 mmol, 89%) der Titelverbindung als farbloses Öl.
IR (Film): 3070, 3040, 2940, 2850, 1740, 1425, 1350, 1255, 1110, 1075, 1035, 1020, 870, 820, 740 und 700 cm⁻¹.

### Beispiel 40:

### 4-{((E)-1S,5S,6S,7R)-7-Hydroxy-6-[(E/Z)-3-(3-chlorphenyl)-ureidoiminomethyl]-bicyclo[3.3.0]oct-3-yliden}-butansäure:

131 mg (311 µmol) der nach Beispiel 40a dargestellten Verbindung verseift man in Analogie zu Beispiel 1 und isoliert nach Aufarbeitung und Reinigung 46 mg (113 µmol, 36%) der Titelverbindung als farblosen Feststoff.
¹H-NMR (CD₃OD): δ= 1,2-1,34(m,1H), 2,05-2,65(m,13H), 3,93-4,04(m,1H), 5,33(m,1H), 7,03(m,1H), 7,2(d,1H), 7,25(t,1H), 7,71(m,1H).

### Beispiel 40a:

### 4-{((E)-1S,5S,6S,7R)-7-Hydroxy-6-[(E/Z)-3-(3-chlorphenyl)-ureidoiminomethyl]-bicyclo[3.3.0]oct-3-yliden}-butansäuremethylester:

128 mg (355 µmol) der nach Beispiel 38b dargestellten Verbindung setzt man in Analogie zu Beispiel 38a unter Verwendung von 4-(3-Chlorphenyl)-semicarbazidhydrochlorid um und isoliert nach Aufarbeitung und Reinigung 131 mg (311 µmol, 88%) der Titelverbindung als farblosen Feststoff.
IR (CHCl₃): 3380, 2950, 1725, 1690, 1590, 1530, 1480, 1430, 1300, 1220, 1095, 1075, 1010, 930, 875 und 670 cm⁻¹.

### Beispiel 41:

### 4-{((Z)-1S,5S,6S,7R)-7-Hydroxy-6-[(E/Z)-3-(3-chlorphenyl)-ureidoiminomethyl]-bicyclo[3.3.0]oct-3-yliden}-butansäure:

86 mg (171 µmol) der nach Beispiel 41a dargestellten Verbindung verseift man in Analogie zu Beispiel 1 und isoliert nach Aufarbeitung und Reinigung 37 mg (91 µmol, 53%) der Titelverbindung als farblosen Feststoff.
¹H-NMR (CD₃OD): δ= 1,2-1,34(m,1H), 2,0-2,56(m,13H), 3,93-4,04(m,1H), 5,31(m,1H), 7,03(m,1H), 7,22(d,1H), 7,25(t,1H), 7,38(m,1H), 7,72(m,1H).

### Beispiel 41a:

### 4-{((Z)-1S,5S,6S,7R)-7-Hydroxy-6-[(E/Z)-3-(3-chlorphenyl)-ureidoiminomethyl]-bicyclo[3.3.0]oct-3-yliden}-butansäuremethylester:

120 mg (358 µmol) der nach Beispiel 39b dargestellten Verbindung setzt man in Analogie zu Beispiel 38a unter Verwendung von 4-(3-Chlorphenyl)-semicarbazidhydrochlorid um und isoliert nach Aufarbeitung und Reinigung 86 mg (171 µmol, 48%) der Titelverbindung als farbloses Öl.
IR (Film): 3600-3000, 3360, 3210, 3110, 2940, 2840, 1725, 1680, 1585, 1530, 1480, 1430, 1300, 1260, 1230, 1190, 1170, 1130, 1095, 1075, 1010, 870, 775, 735 und 680 cm⁻¹.

### Beispiel 42:

### 4-{((E)-1S,5S,6S,7R)-7-Hydroxy-6-[(E/Z)-3-(4-chlorphenyl)-ureidoiminomethyl]-bicyclo[3.3.0]oct-3-yliden}-butansäure:

118 mg (281 µmol) der nach Beispiel 42a dargestellten Verbindung verseift man in Analogie zu Beispiel 1 und isoliert nach Aufarbeitung und Reinigung 44 mg (108 µmol, 39%) der Titelverbindung als farblosen Feststoff.
¹H-NMR (CD₃OD): δ= 1,2-1,34(m,1H), 2,05-2,6(m,13H), 3,93-4,04(m,1H), 5,32(m,1H), 7,2(d,1H), 7,27(d,2H), 7,52(d,2H).

### Beispiel 42a:

### 4-{((E)-1S,5S,6S,7R)-7-Hydroxy-6-[(E/Z)-3-(4-chlorphenyl)-ureidoiminomethyl]-bicyclo[3.3.0]oct-3-yliden}-butansäuremethylester:

128 mg (355 µmol) der nach Beispiel 38b dargestellten Verbindung setzt man in Analogie zu Beispiel 38a unter Verwendung von 4-(4-Chlorphenyl)-semicarbazidhydrochlorid um und isoliert nach Aufarbeitung und Reinigung 118 mg (281 µmol, 79%) der Titelverbindung als farblosen Feststoff.
IR (CHCl₃): 3600-3000, 3380, 3200, 3100, 2950, 1725, 1685, 1590, 1530, 1490, 1435, 1405, 1310, 1220, 1175, 1125, 1090, 1010, 935 und 825 cm⁻¹.

### Beispiel 43:

### 4-{((Z)-1S,5S,6S,7R)-7-Hydroxy-6-[(E/Z)-3-(4-chlorphenyl)-ureidoiminomethyl]-bicyclo[3.3.0]oct-3-yliden}-butansäure:

83 mg (165 µmol) der nach Beispiel 43a dargestellten Verbindung verseift man in Analogie zu Beispiel 1 und isoliert nach Aufarbeitung und Reinigung 42 mg (103 µmol, 63%) der Titelverbindung als farblosen Feststoff.
¹H-NMR (CD₃OD): δ= 1,2-1,34(m,1H), 1,95-2,55(m,13H), 3,93-4,04(m,1H), 5,33(m,1H), 7,22(d,1H), 7,27(d,2H), 7,52(d,2H).

### Beispiel 43a:

### 4-{((Z)-1S,5S,6S,7R)-7-Hydroxy-6-[(E/Z)-3-(4-chlorphenyl)-ureidoiminomethyl]-bicyclo[3.3.0]oct-3-yliden}-butansäuremethylester:

120 mg (358 µmol) der nach Beispiel 39b dargestellten Verbindung setzt man in Analogie zu Beispiel 38a unter Verwendung von 4-(4-Chlorphenyl)-semicarbazidhydrochlorid um und isoliert nach Aufarbeitung und Reinigung 83 mg (165 µmol, 46%) der Titelverbindung als farbloses Öl.
IR (Film): 3600-3000, 3360, 3110, 2940, 2840, 1725, 1680, 1590, 1530, 1490, 1405, 1310, 1230, 1175, 1125, 1090, 1010, 825 und 740 cm⁻¹.

### Beispiel 44:

### 4-{((E)-1S,5S,6S,7R)-7-Hydroxy-6-[(E/Z)-3-(3,4-dichlorphenyl)-ureidoiminomethyl]-bicyclo[3.3.0]oct-3-yliden}-butansäure:

119 mg (262 µmol) der nach Beispiel 44a dargestellten Verbindung verseift man in Analogie zu Beispiel 1 und isoliert nach Aufarbeitung und Reinigung 39 mg (89 µmol, 34%) der Titelverbindung als farblosen Feststoff.
¹H-NMR (CD₃OD): δ= 1,2-1,34(m,1H), 2,05-2,65(m,13H), 3,93-4,03(m,1H), 5,33(m,1H), 7,2(d,1H), 7,42(m,2H), 7,88(m,1H).

### Beispiel 44a:

### 4-{((E)-1S,5S,6S,7R)-7-Hydroxy-6-[(E/Z)-3-(3,4-dichlorphenyl)-ureidoiminomethyl]-bicyclo[3.3.0]oct-3-yliden}-butansäuremethylester:

128 mg (355 µmol) der nach Beispiel 38b dargestellten Verbindung setzt man in Analogie zu Beispiel 38a unter Verwendung von 4-(3,4-Dichlorphenyl)-semicarbazidhydrochlorid um und isoliert nach Aufarbeitung und Reinigung 119 mg (262 µmol, 74%) der Titelverbindung als farblosen Feststoff.
IR (KBr): 3370, 3200, 3110, 2940, 2840, 1735, 1690, 1580, 1530, 1475, 1395, 1300, 1190, 1165, 1120, 1025, 940, 875, 810 und 745 cm⁻¹.

### Beispiel 45:

### 4-{((Z)-1S,5S,6S,7R)-7-Hydroxy-6-[(E/Z)-3-(3,4-dichlorphenyl)-ureidoiminomethyl]-bicyclo[3.3.0]oct-3-yliden}-butansäure:

95 mg (176 µmol) der nach Beispiel 45a dargestellten Verbindung verseift man in Analogie zu Beispiel 1 und isoliert nach Aufarbeitung und Reinigung 46 mg (104 µmol, 59%) der Titelverbindung als farblosen Feststoff.
¹H-NMR (CD₃OD): δ= 1,2-1,34(m,1H), 1,95-2,6(m,13H), 3,94-4,04(m,1H), 5,31(m,1H), 7,22(d,1H), 7,42(m,2H), 7,89(m,1H).

### Beispiel 45a:

### 4-{((Z)-1S,5S,6S,7R)-7-Hydroxy-6-[(E/Z)-3-(3,4-dichlorphenyl)-ureidoiminomethyl]-bicyclo[3.3.0]oct-3-yliden}-butansäuremethylester:

120 mg (358 µmol) der nach Beispiel 39b dargestellten Verbindung setzt man in Analogie zu Beispiel 38a unter Verwendung von 4-(3,4-Dichlorphenyl)-semicarbazidhydrochlorid um und isoliert nach Aufarbeitung und Reinigung 95 mg (176 µmol, 49%) der Titelverbindung als farbloses Öl.
IR (Film): 3600-3000, 3360, 3200, 3110, 2940, 2840, 1730, 1690, 1580, 1530, 1475, 1395, 1295, 1265, 1175, 1120, 1025, 935, 875, 810 und 740 cm⁻¹.

### Beispiel 46:

### 4-{((E)-1S,5S,6S,7R)-7-Hydroxy-6-[(E/Z)-3-(4-nitrophenyl)-ureidoiminomethyl]-bicyclo[3.3.0]oct-3-yliden}-butansäure:

123 mg (286 µmol) der nach Beispiel 46a dargestellten Verbindung verseift man in Analogie zu Beispiel 1 und isoliert nach Aufarbeitung und Reinigung 27 mg (65 µmol, 23%) der Titelverbindung als farblosen Feststoff.
¹H-NMR (CD₃OD): δ= 1,2-1,33(m,1H), 2,05-2,6(m,13H), 3,93-4,04(m,1H), 5,33(m,1H), 7,22(d,1H), 7,8(d,2H), 8,2(d,2H).

### Beispiel 46a:

### 4-{((E)-1S,5S,6S,7R)-7-Hydroxy-6-[(E/Z)-3-(4-nitrophenyl)-ureidoiminomethyl]-bicyclo[3.3.0]oct-3-yliden}-butansäuremethylester:

128 mg (355 µmol) der nach Beispiel 38b dargestellten Verbindung setzt man in Analogie zu Beispiel 38a unter Verwendung von 4-(4-Nitrophenyl)-semicarbazidhydrochlorid um und isoliert nach Aufarbeitung und Reinigung 123 mg (286 µmol, 81%) der Titelverbindung als farblosen Feststoff.
IR (CHCl₃): 3600-3000, 3360, 3210, 2950, 1720, 1695, 1600, 1535, 1510, 1415, 1335, 1220, 1175, 1110 und 850 cm⁻¹.

### Beispiel 47:

### 4-{((Z)-1S,5S,6S,7R)-7-Hydroxy-6-[(E/Z)-3-(4-nitrophenyl)-ureidoiminomethyl]-bicyclo[3.3.0]oct-3-yliden}-butansäure:

93 mg (181 µmol) der nach Beispiel 47a dargestellten Verbindung verseift man in Analogie zu Beispiel 1 und isoliert nach Aufarbeitung und Reinigung 36 mg (86 µmol, 48%) der Titelverbindung als farblosen Feststoff.
¹H-NMR (CD₃OD): δ= 1,2-1,32(m,1H), 1,98-2,58(m,13H), 3,98(m,1H), 5,32(m,1H), 7,24(d,1H), 7,8(d,2H), 8,18(d,2H).

### Beispiel 47a:

### 4-{((Z)-1S,5S,6S,7R)-7-Hydroxy-6-[(E/Z)-3-(4-nitrophenyl)-ureidoiminomethyl]-bicyclo[3.3.0]oct-3-yliden}-butansäuremethylester:

120 mg (358 µmol) der nach Beispiel 39b dargestellten Verbindung setzt man in Analogie zu Beispiel 38a unter Verwendung von 4-(4-Nitrophenyl)-semicarbazidhydrochlorid um und isoliert nach Aufarbeitung und Reinigung 93 mg (181 µmol, 51%) der Titelverbindung als farbloses Öl.
IR (Film): 3600-3000, 3340, 3110, 2940, 2840, 1720, 1705, 1690, 1600, 1540, 1505, 1415, 1330, 1235, 1175, 1110, 1000, 850 und 735 cm⁻¹.

### Beispiel 48:

### 5-{((Z)-1S,5S,6S,7R)-7-Hydroxy-6-[(E/Z)-2-(2.4.5-trichlorphenylsulfonyl)-hydrazonomethyl]-bicyclo[3.3.0]oct-3-yliden}pentansäure:

56 mg (107 µmol) der nach Beispiel 48a dargestellten Verbindung verseift man in Analogie zu Beispiel 1 und isoliert nach Aufarbeitung und Reinigung 30 mg (59 µmol, 55%) der Titelverbindung als farbloses Öl.
¹H-NMR (CD₃OD): δ= 1,1-1,21(m,1H), 1,52-2,54(m,14H), 3,8-3,9(m,1H), 5,18-5,25(m,1H), 7,29(d,1H), 7,88(m,1H), 8,20(m,1H).

### Beispiel 48a:

### 5-{((Z)-1S,5S,6S,7R)-7-Hydroxy-6-[(E/Z)-2-(2.4.5-trichlorphenylsulfonyl)-hydrazonomethyl]-bicyclo[3.3.0]oct-3-yliden}pentansäuremethylester:

Die Lösung von 99 mg (283 µmol) des nach Beispiel 2c dargestellten Aldehyds in 2,5 ml wasserfreiem Ethanol versetzt man mit 106 mg 2.4.5-Trichlorphenylsulfonsäurehydrazid und rührt 1,5 Stunden bei 55°C unter einer Atmosphäre aus trockenem Argon. Danach versetzt man mit einer Spatelspitze Pyridinium-p-toluolsulfonat und erhitzt weitere 2 Stunden auf 55°C. Den nach Lösungsmittelabzug erhaltenen Rückstand reinigt man durch Chromatographie an 7 analytischen Dünnschichtplatten. Als Laufmittel dient ein Gemisch aus n-Hexan und Ethylacetat, als Elutionsmittel Trichlormethan und Ethanol. Isoliert werden 56 mg (107 µmol, 38%) der Titelverbindung als farbloses Öl.
IR (Film): 3600-3100, 3200, 3090, 2840, 1730, 1710, 1440, 1365, 1325, 1170, 1065, 870, 735, 690, 660 und 625 cm⁻¹.

### Beispiel 49:

### 5-{((Z)-1S,5S,6S,7R)-7-Hydroxy-6-[(E/Z)-2-(4-methoxyphenylsulfonyl)-hydrazonomethyl] -bicyclo[3.3.0]oct-3-yliden}pentansäure:

87 mg (193 µmol) der nach Beispiel 49a dargestellten Verbindung verseift man in Analogie zu Beispiel 1 und isoliert nach Aufarbeitung und Reinigung 44 mg (101 µmol, 52%) der Titelverbindung als farbloses Öl.
¹H-NMR (CD₃OD): δ= 1,13-1,26(m,1H), 1,6-1,72(m,2H), 1,9-2,55(m,12H), 3,8-3,9(m,1H), 3,87(s,3H), 5,18-5,29(m,1H), 7,07(d,2H), 7,18(d,1H), 7,81(d,2H).

### Beispiel 49a:

### 5-{((Z)-1S,5S,6S,7R)-7-Hydroxy-6-[(E/Z)-2-(4-methoxyphenylsulfonyl)-hydrazonomethyl] -bicyclo[3.3.0]oct-3-yliden}pentansäuremethylester:

99 mg (283 µmol) der nach Beispiel 2c dargestellten Verbindung setzt man in Analogie zu Beispiel 48a unter Verwendung von (4-Methoxyphenyl)-sulfonsäurehydrazid um und isoliert nach Aufarbeitung und Reinigung 87 mg (193 µmol, 68%) der Titelverbindung als farbloses Öl.
IR (Film): 3600-3000, 3200, 2940, 2840, 1735, 1595, 1495, 1435, 1350, 1320, 1260, 1155, 1095, 1020, 935, 835, 800, 735 und 675 cm⁻¹.

### Beispiel 50:

### 5-{((Z)-1S,5S,6S,7R)-7-Hydroxy-6-[(E/Z)-2-(4-fluorphenylsulfonyl)-hydrazonomethyl]-bicyclo[3.3.0]oct-3-yliden}pentansäure:

50 mg (114 µmol) der nach Beispiel 50a dargestellten Verbindung verseift man in Analogie zu Beispiel 1 und isoliert nach Aufarbeitung und Reinigung 27 mg (64 µmol, 56%) der Titelverbindung als farbloses Öl.
¹H-NMR (CD₃OD): δ= 1,13-1,25(m,1H), 1,6-1,73(m,2H), 1,9-2,55(m,12H), 3,8-3,9(m,1H), 5,2-5,3(m,1H), 7,18(d,1H), 7,24-7,34(m,2H), 7,88-7,98(m,2H).

### Beispiel 50a:

### 5-{((Z)-1S,5S,6S,7R)-7-Hydroxy-6-[(E/Z)-2-(4-fluorphenylsulfonyl)-hydrazonomethyl]-bicyclo[3.3.0]oct-3-yliden}pentansäuremethylester:

99 mg (283 µmol) der nach Beispiel 2c dargestellten Verbindung setzt man in Analogie zu Beispiel 48a unter Verwendung von (4-Fluorphenyl)-sulfonsäurehydrazid um und isoliert nach Aufarbeitung und Reinigung 50 mg (114 µmol, 40%) der Titelverbindung als farbloses Öl.
IR (Film): 3600-3250, 3200, 3060, 2940, 1730, 1590, 1490, 1435, 1355, 1320, 1265, 1240, 1170, 1155, 1090, 840, 735, 700 und 670 cm⁻¹.

### Beispiel 51:

### 5-{((Z)-1S,5S,6S,7R)-7-Hydroxy-6-[(E/Z)-2-(4-methylphenylsulfonyl)-hydrazonomethyl]-bicyclo[3.3.0]oct-3-yliden}pentansäure:

69 mg (159 µmol) der nach Beispiel 51a dargestellten Verbindung verseift man in Analogie zu Beispiel 1 und isoliert nach Aufarbeitung und Reinigung 32 mg (76 µmol, 48%) der Titelverbindung als farbloses Öl.
¹H-NMR (CD₃OD): δ= 1,12-1,24(m,1H), 1,6-1,73(m,2H), 1,88-2,54(m,12H), 2,42(s,3H), 3,8-3,9(m,1H), 5,2-5,29(m,1H), 7,17(d,1H), 7,38(d,2H), 7,76(m,2H).

### Beispiel 51a:

### 5-{((Z)-1S,5S,6S,7R)-7-Hydroxy-6-[(E/Z)-2-(4-methylphenylsulfonyl)-hydrazonomethyl]-bicyclo[3.3.0]oct-3-yliden}pentansäuremethylester:

69 mg (197 µmol) der nach Beispiel 2c dargestellten Verbindung setzt man in Analogie zu Beispiel 48a unter Verwendung von Toluolsulfonsäurehydrazid um und isoliert nach Aufarbeitung und Reinigung 69 mg (159 µmol, 81%) der Titelverbindung als farbloses Öl.
IR (CHCl₃): 3600-3300, 3180, 3000, 2950, 2860, 1725, 1595, 1435, 1355, 1325, 1255, 1165, 1090, 1035, 1020, 810 und 660 cm⁻¹.

### Beispiel 52:

### 5-{((Z)-1S,5S,6S,7R)-7-Hydroxy-6-[(E/Z)-2-(phenylsulfonyl)-hydrazonomethyl]-bicyclo[3.3.0]oct-3-yliden}pentansäure:

59 mg (140 µmol) der nach Beispiel 52a dargestellten Verbindung verseift man in Analogie zu Beispiel 1 und isoliert nach Aufarbeitung und Reinigung 30 mg (74 µmol, 53%) der Titelverbindung als farbloses Öl.
¹H-NMR (CD₃OD): δ= 1,12-1,25(m,1H), 1,6-1,72(m,2H), 1,88-2,54(m,12H), 3,79-3,9(m,1H), 5,2-5,28(m,1H), 7,17(d,1H), 7,52-7,68(m,3H), 7,85-7,93(m,2H).

### Beispiel 52a:

### 5-{((Z)-1S,5S,6S,7R)-7-Hydroxy-6-[(E/Z)-2-(phenylsulfonyl)-hydrazonomethyl]-bicyclo[3.3.0]oct-3-yliden}pentansäuremethylester:

69 mg (197 µmol) der nach Beispiel 2c dargestellten Verbindung setzt man in Analogie zu Beispiel 48a unter Verwendung von Benzolsulfonsäurehydrazid um und isoliert nach Aufarbeitung und Reinigung 59 mg (140 µmol, 71%) der Titelverbindung als farbloses Öl.
IR (CHCl₃): 3600-3300, 3200, 3000, 2950, 1725, 1630, 1445, 1360, 1320, 1260, 1165, 1090, 1015, 810 und 685 cm⁻¹.

## Patentansprüche

1. Bicyclo[3.3.0]octan-Derivate der Formel I, 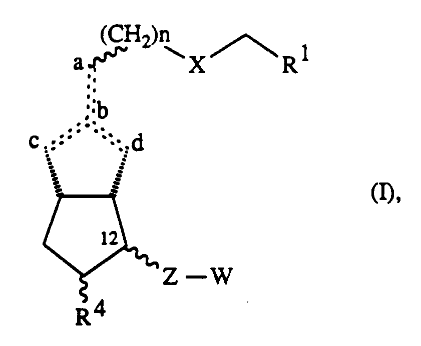 sowie deren Enantiomere,
worin
zwischen den Kohlenstoffatomen der Zentren a-b oder b-c oder b-d maximal eine Doppelbindung liegt, 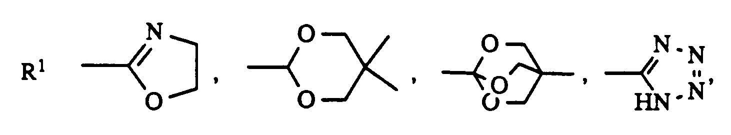 COOR⁵, wobei R⁵
Wasserstoff oder gegebenenfalls durch Halogen, Phenyl, C₁-C₄-Alkoxy oder Di-(C₁-C₄)-alkylamino substituiertes C₁-C₁₀-Alkyl, C₃-C₁₀-Cycloalkyl, C₇-C₁₆-Aralkyl, durch Y substituiertes Phenacyl oder C₆-C₁₂-Aryl oder einen 5- oder 6- gliedrigen heterocyclischen Rest mit wenigstens einem N-, O- oder S-Atom bedeuten kann, oder -CONHR⁷ mit R⁷ in der Bedeutung Wasserstoff, C₁-C₁₀-Alkanoyl oder C₁-C₁₀-Alkansulfonyl sein kann,
X eine CH₂-Gruppe oder ein O- oder S-Atom,
n 0-2,
R⁴ ein Wasserstoffatom, eine freie oder funktionell abgewandelte Hydroxygruppe, wobei die OH-Gruppe α- oder β-ständig sein kann, 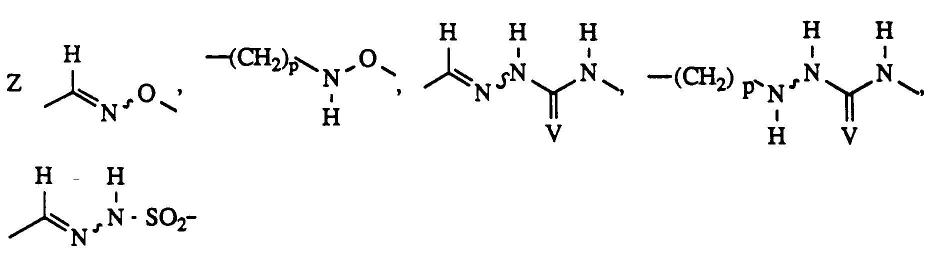 p 0 oder 1,
V ein O- oder S-Atom,
W durch Y substituiertes C₁-C₁₀-Alkyl, C₃-C₁₀-Cycloalkyl, 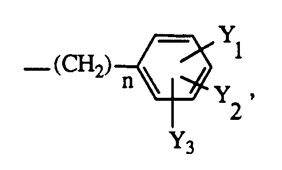 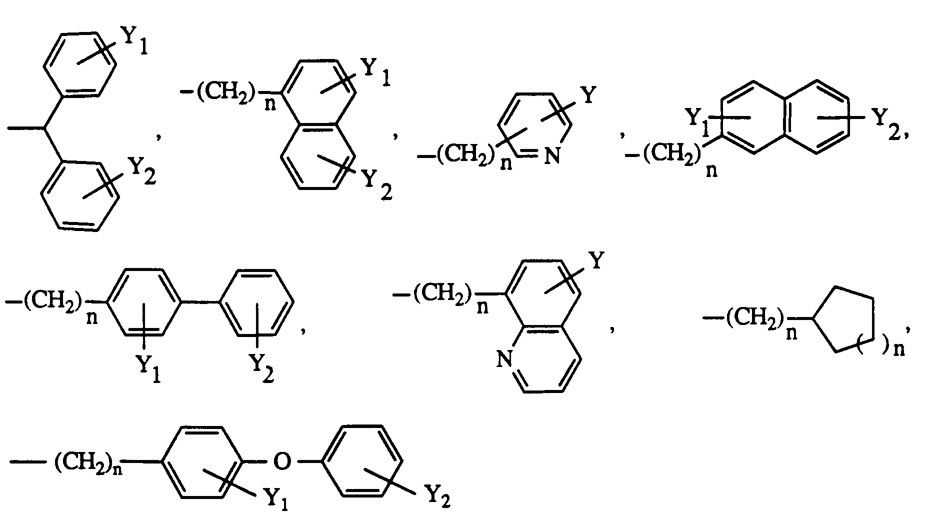 Y_{1,} Y₂ und Y₃ gleich oder verschieden sind und Y bedeuten,
Y Wasserstoff, Halogen, N₃, CF₃, OR⁶, NO₂, COOR⁶ oder C₁-C₁₀-Alkyl,
R⁶ Wasserstoff, C₁-C₁₀-Alkyl, gegebenenfalls durch Halogen substituiertes C₆-C₁₂-Aryl oder C₇-C₁₆-Aralkyl sein kann und, falls R⁵ Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.

2. Verfahren zur Herstellung der Verbindungen der Formel I, dadurch gekennzeichnet, daß man eine Verbindung der Formel II 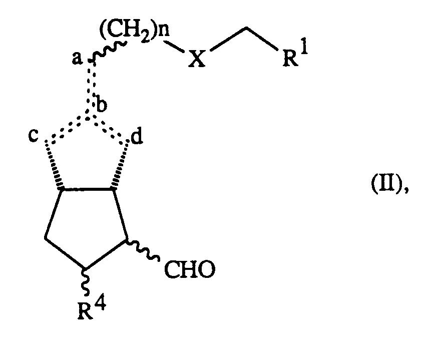 worin R¹, X, n, a-b, b-c, b-d und R⁴ die oben angegebenen Bedeutungen aufweisen und freie OH-Gruppen in R⁴ und W geschützt sind, mit Aminoverbindungen der Formel 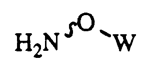 oder 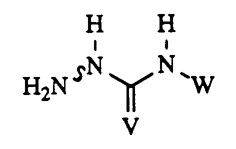 oder H₂N-NH-SO₂-W worin V die oben angegebene Bedeutung hat und freie OH-Gruppen in W geschützt sind, umsetzt und geschützte Hydroxygruppen in R⁴ und W freisetzt und/oder freie Hydroxygruppen verestert, verethert und/oder eine veresterte Carboxygruppe verseift oder eine Carboxylgruppe mit einer physiologisch vertraglichen Base in ein Salz überführt oder mit α-, β- oder γ-Cyclodextrin zu einem Clathrat umsetzt oder mit Liposomen verkapselt.

3. Hilfsstoffe für pharmakologische Untersuchungen und Arzneimittel aus einer oder mehreren Verbindungen der Formel I und üblichen Hilfs-, Träger- und Zusatzstoffen.

## Claims

1. Bicyclo[3.3.0]octane derivatives of the formula I 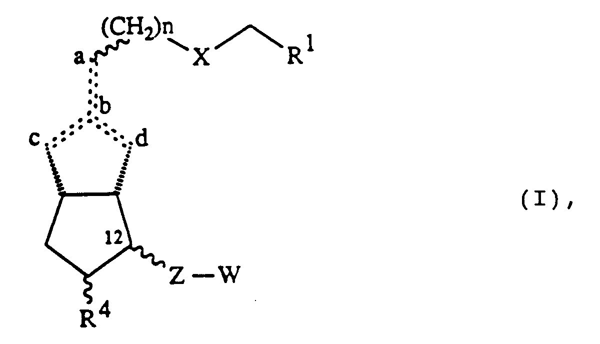 and enantiomers thereof,
wherein
between the carbon atoms of the centres a-b or b-c or b-d there is a maximum of one double bond,
R₁ may be 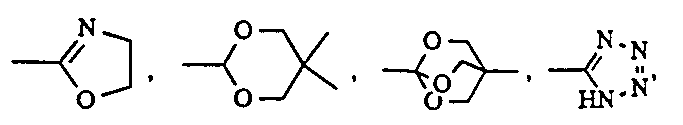 COOR⁵
wherein R⁵ may represent hydrogen or optionally halo-, phenyl-, C₁-C₄-alkoxy- or di-(C₁-C₄)-alkylamino-substituted C₁-C₁₀-alkyl, C₃-C₁₀-cycloalkyl, C₇-C₁₆-aralkyl, Y-substituted phenacyl or C₆-C₁₂-aryl or a 5- or 6-membered heterocyclic radical having at least one N, O or S atom,
or may be -CONHR⁷ wherein R⁷ may be hydrogen, C₁-C₁₀-alkanoyl or C₁-C₁₀-alkanesulphonyl,
X represents a CH₂ group or an O or S atom,
n represents 0-2,
R⁴ represents a hydrogen atom, a free or functionally modified hydroxy group, wherein the OH group may be in the α- or β-configuration,
Z represents 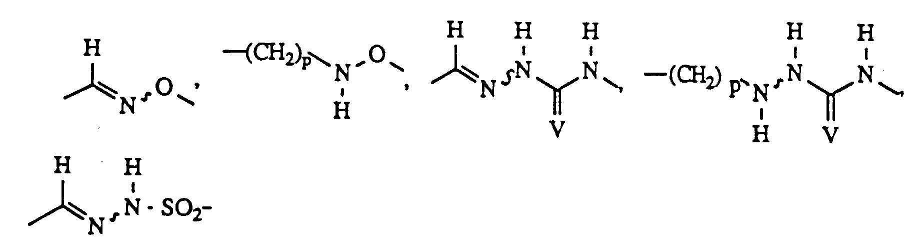 p represents 0 or 1
V represents an O or S atom,
W represents Y-substituted C₁-C₁₀-alkyl, C₃-C₁₀-cycloalkyl, 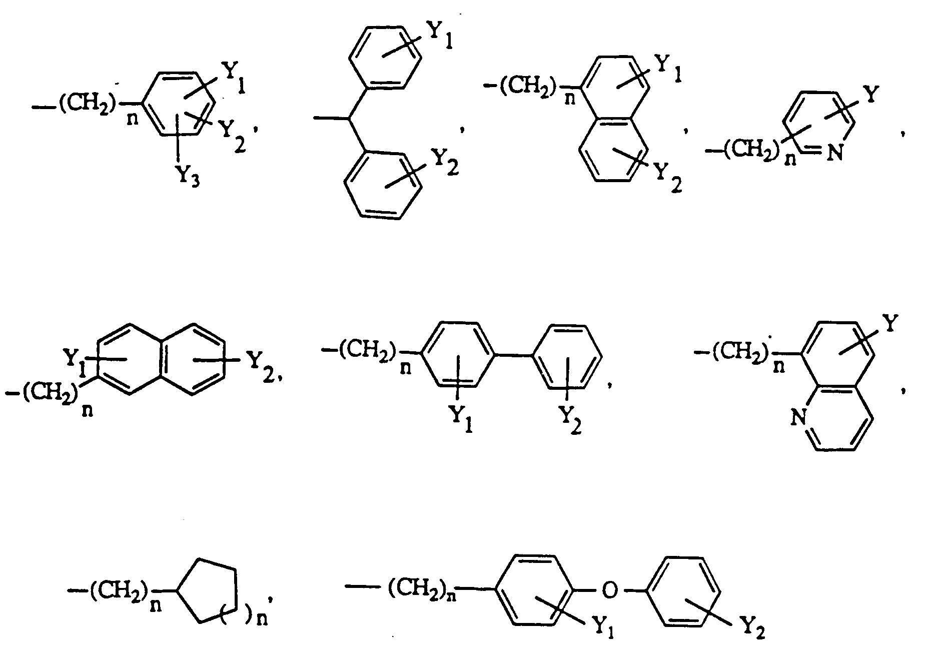 Y₁, Y₂ and Y₃ are identical or different and represent Y,
Y may be hydrogen, halogen, N₃, CF₃, OR⁶, NO₂, COOR⁶ or C₁-C₁₀-alkyl,
R₆ may be hydrogen, C₁-C₁₀-alkyl, optionally halo-substituted C₆-C₁₂-aryl or C₇-C₁₆-aralkyl,
and, when R⁵ is hydrogen, the salts thereof with physiologically tolerable bases, and the α-, β or γ-cyclodextrin clathrates and the liposome-encapsulated compounds of the formula I.

2. Process for the manufacture of the compounds of the formula I, characterised in that a compound of the formula II 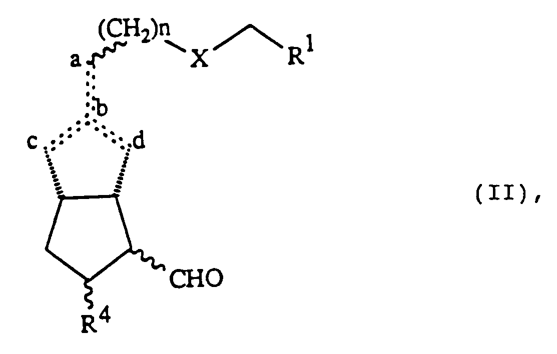 wherein R¹, X, n, a-b, b-c, b-d and R⁴ have the meanings given above and free OH groups in R⁴ and W are protected, is reacted with amino compounds of the formula 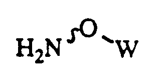 or 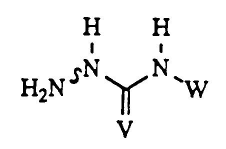 or H₂N-NH-SO₂-W wherein V has the meanings given above and free OH groups in W are protected, and protected hydroxy groups in R⁴ and W are freed and/or free hydroxy groups are esterified, etherified and/or an esterified carboxy group is hydrolysed or a carboxy group is converted into a salt with a physiologically tolerable base or is reacted with α-, β- or γ-cyclodextrin to form a clathrate or is encapsulated with liposomes.

3. Adjuvants for pharmacological investigations, and medicaments consisting of one or more compounds of the formula I and conventional adjuvants, carriers and additives.

## Revendications

1. Dérivés de bicyclo[3.3.0]octane de formule I, 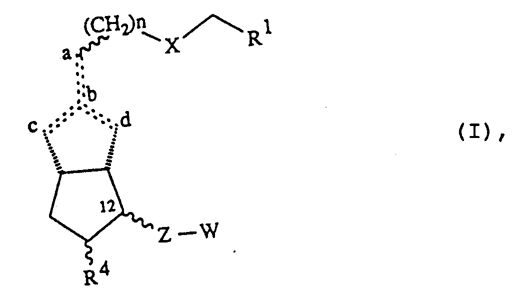 ainsi que leurs énantiomères,
formule dans laquelle,
entre les atomes de carbone des centres a-b, b-c ou b-d, il y a au maximum une double liaison,
R¹ peut représenter 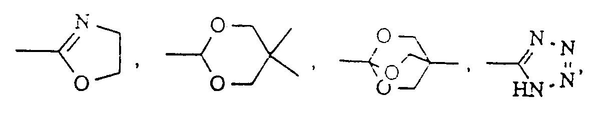 COOR⁵, R⁵ désignant l'hydrogène, un alkyle en C₁-C₁₀, un cycloalkyle en C₃-C₁₀, un aralkyle en C₇-C₁₆, éventuellement substitué par des halogène, phényle, alcoxy en C₁-C₄ ou dialkyl(en C₁-C₄)amino, un phénacyle ou un aryle en C₆-C₁₂ substitué par Y, ou un radical hétérocyclique à 5 ou 6 chaînons ayant au moins un atome d'azote, d'oxygène ou de soufre, ou bien peut être -CONHR⁷, R⁷ pouvant désigner l'hydrogène, un alcanoyle en C₁-C₁₀ ou un (alcane en C₁-C₁₀)- sulfonyle,
X représente un groupe CH₂ ou un atome d'oxygène ou de soufre,
n vaut de 0 à 2,
R⁴ un atome d'hydrogène ou un groupe hydroxylique libre ou fonctionnellement modifié, le groupe OH pouvant occuper la position α ou β,
Z représente 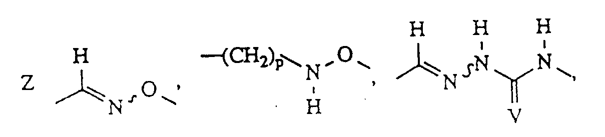 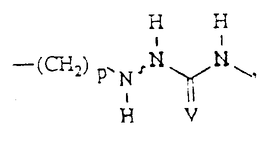 ou 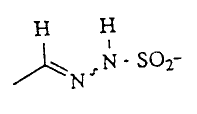 p vaut 0 ou 1 et
V signifie un atome d'oxygène ou de soufre,
W représente, substitué par Y, un alkyle en C₁-C₁₀, un cycloalkyle en C₃-C₁₀, 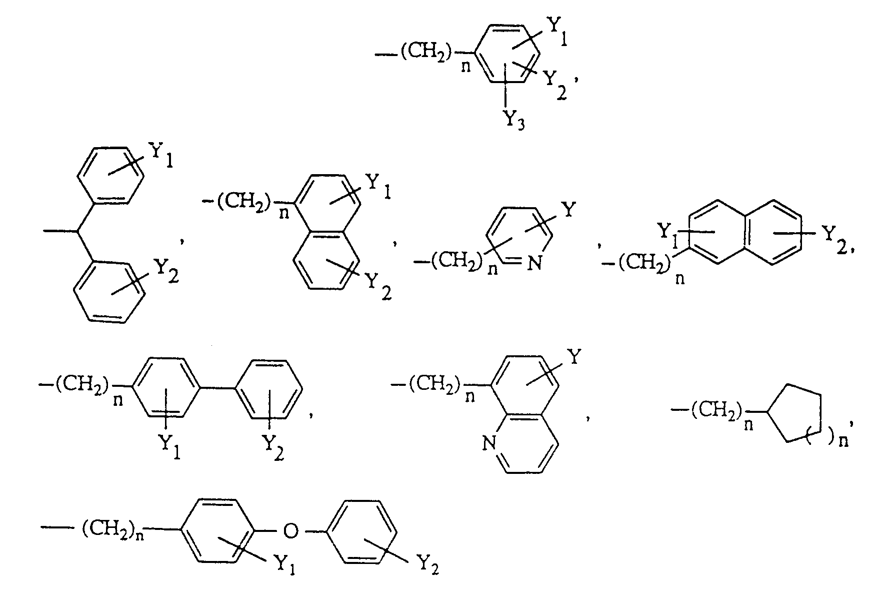 Y₁, Y₂ et Y₃ sont identiques ou différents les uns des autres et désignent Y,
Y signifie l'hydrogène, un halogène, N₃, CF₃, OR⁶, NO₂, COOR⁶ ou un alkyle en C₁-C₁₀,
R⁶ peut être l'hydrogène, un alkyle en C₁-C₁₀, un aryle en C₆-C₁₂ ou aralkyle en C₇-C₁₆, éventuellement halogéné, et, si R⁵ est l'hydrogène, des sels de ces dérivés avec des bases physiologiquement compatibles, ainsi que les clathrates de cyclodextrines α, β ou γ et les composés de formule I encapsulés avec des liposomes.

2. Procédé de préparation des composés de formule I, caractérisé en ce que l'on fait réagir un composé de formule II 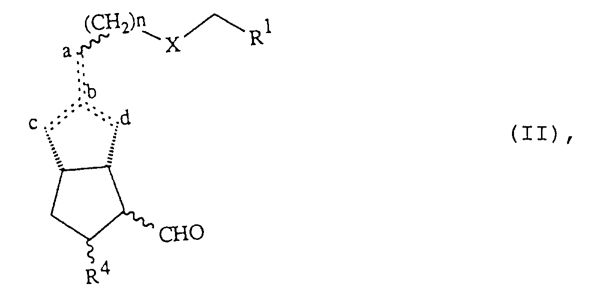 dans lequel R¹, x, n, a-b, b-c, b-d et R⁴ possèdent les significations données ci-dessus et où des groupes OH libres de R⁴ et W sont protégés,
avec des composés aminés de formule 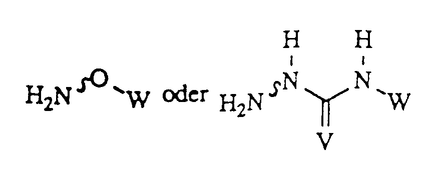 ou
H₂N-NH-SO₂-W dans lesquelles V a la signification donnée ci-dessus et où des groupes OH libres de W sont protégés, puis on libère des hydroxyles protégés de R⁴ et W et/ou on estérifie ou éthérifie des groupes hydroxy libres et/ou on saponifie un groupe carboxy estérifié ou on salifie un groupe carboxyle avec une base physiologiquement compatible ou on fait réagir les composés obtenus avec une cyclodextrine α, β ou γ pour en former un clathrate ou on les encapsule avec des liposomes.

3. Adjuvants pour investigations pharmacologiques et médicaments formés d'un ou de plusieurs composés de formule I avec des excipients, des supports ou véhicules et des additifs habituels.
